# EUROPEAN PATENT SPECIFICATION

(11) **EP 4 255 284 B1**
(45) Date of publication and mention of the grant of the patent: **03.12.2025**
(21) Application number: 21798234.7
(22) Date of filing: 30.09.2021
(51) Int. Cl.: A61B 5/00, A61B 5/021, A61B 5/0285

(54) **NON-INVASIVE BLOOD PRESSURE ESTIMATION AND BLOOD VESSEL MONITORING BASED ON PHOTOACOUSTIC PLETHYSMOGRAPHY**
NICHTINVASIVE BLUTDRUCKSCHÄTZUNG UND BLUTGEFÄSSÜBERWACHUNG AUF BASIS VON PHOTOAKUSTISCHER PLETHYSMOGRAFIE
ESTIMATION NON INVASIVE DE LA TENSION ARTÉRIELLE ET SURVEILLANCE DE VAISSEAU SANGUIN BASÉE SUR UNE PLÉTHYSMOGRAPHIE PHOTOACOUSTIQUE

(30) Priority: 07.12.2020 US 202017247323
(43) Date of publication of application: 11.10.2023
(73) Proprietor: QUALCOMM INCORPORATED, San Diego, California 92121-1714 (US)
(72) Inventor: KITCHENS, Jack Conway, San Diego, California 92121-1714 (US); SCHNEIDER, John Keith, San Diego, California 92121-1714 (US); BRELOFF, Evan Michael, San Diego, California 92121-1714 (US); BROOKS, Emily Kathryn, San Diego, California 92121-1714 (US); GOJEVIC, Stephen Michael, San Diego, California 92121-1714 (US); JOHN ARCHIBALD, Fitzgerald, San Diego, California 92121-1714 (US); STOIANOV, Alexei, San Diego, California 92121-1714 (US); GORE, Shounak Uday, San Diego, California 92121-1714 (US); BUCHAN, Nicholas Ian, San Diego, California 92121-1714 (US)
(74) Representative: Reddie & Grose LLP
(86) International application number: PCT/US2021/052835
(87) International publication number: WO 2022/125171

(56) References cited:
- WO-A1-2013/132977
- CN-A- 101 171 046
- US-A1- 2013 190 589
- US-A1- 2015 297 091
- ZHANG XIAOMAN ET AL: "Photoacoustic identification of blood vessel deformation under pressure", AIP ADVANCES, AMERICAN INSTITUTE OF PHYSICS, 2 HUNTINGTON QUADRANGLE, MELVILLE, NY 11747, vol. 9, no. 7, 24 July 2019 (2019-07-24), XP012239341, DOI: 10.1063/1.5108852
- WILFRIED GYSELAERS ET AL: "Maternal venous hemodynamics in gestational hypertension and preeclampsia", BMC PREGNANCY AND CHILDBIRTH, BIOMED CENTRAL LTD., LONDON, GB, vol. 14, no. 1, 23 June 2014 (2014-06-23), pages 212, XP021190187, ISSN: 1471-2393, DOI: 10.1186/1471-2393-14-212

## Description

### TECHNICAL FIELD

This disclosure relates generally to non-invasive blood pressure estimation and blood vessel monitoring.

### DESCRIPTION OF RELATED TECHNOLOGY

A variety of different sensing technologies and algorithms are being investigated for use in various biomedical applications, including health and wellness monitoring. This push is partly a result of the limitations in the usability of traditional measuring devices for continuous, noninvasive and ambulatory monitoring. For example, a sphygmomanometer is an example of a traditional blood pressure monitoring device that utilizes an inflatable cuff to apply a counter pressure to a region of interest (for example, around an upper arm of a subject). The pressure exerted by the inflatable cuff is designed to restrict arterial flow in order to provide a measurement of systolic and diastolic pressure. Such traditional sphygmomanometers inherently affect the physiological state of the subject, which can introduce an error in the blood pressure measurements. Such sphygmomanometers also can affect the psychological state of the subject, which can manifest itself in a physiological state change, and thus, introduce an error in the blood pressure measurements. For example, such devices are often used primarily on isolated occasions, for example, when a subject visits a doctor's office or is being treated in a hospital setting. Naturally, some subjects experience anxiety during such occasions, and this anxiety can influence (for example, increase) the user's blood pressure as well as heart rate.

For these and other reasons, such devices may not provide an accurate estimation or "picture" of blood pressure, and a user's health in general, over time. While implanted or otherwise invasive devices may provide better estimates of blood pressure over time, such invasive devices generally involve greater risk than noninvasive devices and are generally not suitable for ambulatory use.

US2015297091 describes a photoacoustic imaging device, configured to detect whether more oxidized hemoglobin or more reduced hemoglobin is contained in blood, which can be used for distinguishing arteries and veins from each other.

US 2013/190589 A1 discloses multiple peak analysis in a photoacoustic system. A first wavelength photonic signal may generate a first photoacoustic signal and a second wavelength photonic signal may generate a second photoacoustic signal. These measurements may be made concurrently or consecutively in an alternating fashion. Concurrently measured signals at multiple wavelengths may be measured at spatially separated locations. Emitting photonic signals and detecting acoustic pressure signals may be repeated multiple times. For example, a measurement as described herein may be carried out 5 times at a first wavelength and 5 times at a second wavelength. The measurements may take place within one cardiac pulse cycle or over several cardiac pulse cycles such that the physiological parameters remain relatively constant. The system may average measurements over, for example, seconds, minutes or hours.

ZHANG XIAOMAN ET AL: "Photoacoustic identification of blood vessel deformation under pressure", AIP ADVANCES, AMERICAN INSTITUTE OF PHYSICS, 2 HUNTINGTON QUADRANGLE, MELVILLE, NY 11747, vol. 9, no. 7, 24 July 2019 (2019-07-24), demonstrates a photoacoustic system which can identify the deformation of blood vessels under external pressure. Using photoacoustic imaging method, the vessel internal diameter can be derived from the peak-to-peak time interval of the laser (532nm) induced photoacoustic signals.

### SUMMARY

The systems, methods and devices of this disclosure each have several aspects, no single one of which is solely responsible for the desirable attributes disclosed herein.

The invention is defined in the independent method claims. Optional features are set out in the dependent claims. One innovative aspect of the subject matter described in this disclosure can be implemented in an apparatus, or in a system that includes the apparatus. In some implementations, a mobile device (such as a wearable device) may be, or may include, at least part of the apparatus.

The control system may include one or more general purpose single- or multi-chip processors, digital signal processors (DSPs), application specific integrated circuits (ASICs), field programmable gate arrays (FPGAs) or other programmable logic devices, discrete gates or transistor logic, discrete hardware components, or combinations thereof. The control system may be configured for controlling the light source system to emit a plurality of light pulses into biological tissue at the pulse repetition frequency. The biological tissue may, for example, include blood and blood vessels at depths within the biological tissue.

Other innovative aspects of the subject matter described in this disclosure can be implemented in a method, such as a biometric method. [0020] Some or all of the methods described herein may be performed by one or more devices according to instructions (e.g., software) stored on non-transitory media. Such non-transitory media may include memory devices such as those described herein, including but not limited to random access memory (RAM) devices, read-only memory (ROM) devices, etc. Accordingly, some innovative aspects of the subject matter described in this disclosure can be implemented in one or more non-transitory media having software stored thereon. The software may include instructions for controlling one or more devices to perform one or more disclosed methods.

Details of one or more implementations of the subject matter described in this disclosure are set forth in the accompanying drawings and the description below. Other features, aspects, and advantages will become apparent from the description, the drawings and the claims. Note that the relative dimensions of the following figures may not be drawn to scale.

### BRIEF DESCRIPTION OF THE DRAWINGS

Figure 1A shows a plot of a blood pressure signal in an example artery versus time during an example cardiac cycle.
Figure 1B shows an example of a blood pressure monitoring device based on photoplethysmography (PPG).
Figure 1C shows an example of two superimposed graphs of blood pressure variation during cardiac cycles.
Figure 1D shows an example of a blood pressure monitoring device based on photoacoustic plethysmography, which may be referred to herein as PAPG.
Figure 2 is a block diagram that shows example components of an apparatus according to some disclosed implementations.
Figure 3 is a flow diagram that provides examples of some disclosed operations.
Figure 4A shows an example of a range-gate window (RGW) selected to receive acoustic waves emitted from a range of different depths.
Figure 4B shows examples of multiple acquisition time delays being selected to receive acoustic waves emitted from different depths.
Figures 5A and 5B shows examples of an apparatus configured to receive acoustic waves emitted from different depths.
Figure 6 shows an example of a cross-sectional view of an apparatus capable of performing the method of Figure 3.
Figure 7 shows examples of vein heart rate waveforms and artery heart rate waveforms.
Figure 8 shows examples of determining vein heart rate waveforms and artery heart rate waveforms.
Figure 9 is a diagram that represents aspects of five disclosed PAPG algorithms.
Figures 10A and 10B show examples of blocks of a data collection and heart rate waveform (HRW) determination process.
Figure 11 shows examples of blocks of some disclosed methods.
Figure 12 shows examples of heart rate waveform (HRW) features that may be extracted according to some implementations of the method of Figure 11.
Figure 13 is a flow diagram that shows example blocks of making blood pressure estimates based on hemodynamic features.
Figure 14 is a flow diagram that provides an example of making a blood pressure estimation that is based on both an HRW analysis and a hemodynamic analysis.
Figure 15 shows examples of devices that may be used in a system for estimating blood pressure based, at least in part, on pulse transit time (PTT).
Figure 16 shows a cross-sectional side view of a diagrammatic representation of a portion of an artery 1600 through which a pulse 1602 is propagating.
Figure 17A shows an example ambulatory monitoring device 1700 designed to be worn around a wrist according to some implementations.
Figure 17B shows an example ambulatory monitoring device 1700 designed to be worn around a finger according to some implementations.
Figure 17C shows an example ambulatory monitoring device 1700 designed to reside on an earbud according to some implementations.
Like reference numbers and designations in the various drawings indicate like elements.

### DETAILED DESCRIPTION

The following description is directed to certain implementations for the purposes of describing various aspects of this disclosure. However, a person having ordinary skill in the art will readily recognize that the teachings herein can be applied in a multitude of different ways. Some of the concepts and examples provided in this disclosure are especially applicable to blood pressure monitoring applications. However, some implementations also may be applicable to other types of biological sensing applications, as well as to other fluid flow systems. The described implementations may be implemented in any device, apparatus, or system that includes an apparatus as disclosed herein. In addition, it is contemplated that the described implementations may be included in or associated with a variety of electronic devices such as, but not limited to: mobile telephones, multimedia Internet enabled cellular telephones, mobile television receivers, wireless devices, smartphones, smart cards, wearable devices such as bracelets, armbands, wristbands, rings, headbands, patches, etc., Bluetooth^{®} devices, personal data assistants (PDAs), wireless electronic mail receivers, hand-held or portable computers, netbooks, notebooks, smartbooks, tablets, printers, copiers, scanners, facsimile devices, global positioning system (GPS) receivers/navigators, cameras, digital media players, game consoles, wrist watches, clocks, calculators, television monitors, flat panel displays, electronic reading devices (e.g., e-readers), mobile health devices, computer monitors, auto displays (including odometer and speedometer displays, etc.), cockpit controls and/or displays, camera view displays (such as the display of a rear view camera in a vehicle), architectural structures, microwaves, refrigerators, stereo systems, cassette recorders or players, DVD players, CD players, VCRs, radios, portable memory chips, washers, dryers, washer/dryers, parking meters, automobile doors, autonomous or semi-autonomous vehicles, drones, Internet of Things (IoT) devices, etc. Thus, the teachings are not intended to be limited to the specific implementations depicted and described with reference to the drawings; rather, the teachings have wide applicability as will be readily apparent to persons having ordinary skill in the art.

Also of note, the conjunction "or" as used herein is intended in the inclusive sense where appropriate unless otherwise indicated; that is, the phrase "A, B or C" is intended to include the possibilities of A individually; B individually; C individually; A and B and not C; B and C and not A; A and C and not B; and A and B and C. Similarly, a phrase referring to "at least one of" a list of items refers to any combination of those items, including single members. As an example, the phrase "at least one of A, B, or C" is intended to cover the possibilities of at least one of A; at least one of B; at least one of C; at least one of A and at least one of B; at least one of B and at least one of C; at least one of A and at least one of C; and at least one of A, at least one of B and at least one of C.

Particular implementations of the subject matter described in this disclosure can be implemented to realize one or more of the following potential advantages. Some implementations of the portable monitoring devices described herein also are designed to consume relatively little power, enabling continuous wearing and monitoring of a biological signal of interest, such as blood pressure, over extended durations of time (for example, hours, days, weeks or even a month or more) without external calibration, recharging or other interruption. Continuous monitoring provides greater prognostic and diagnostic value than isolated measurements, for example, obtained in a hospital or doctor's office setting. Some implementations of the portable or "ambulatory" monitoring devices described herein also are designed with small form factors and with housings that can be coupled to a subject (also referred to herein as a "patient," "person" or "user") so as to be wearable, noninvasive, and nonrestrictive of ambulatory use. In other words, some implementations of the ambulatory monitoring devices described herein do not restrict the free uninhibited motion of a subject's arms or legs enabling continuous or periodic monitoring of cardiovascular characteristics such as blood pressure even as the subject is mobile or otherwise engaged in a physical activity. Not only do such devices not interfere with the subject's daily or other desired activities, they also may encourage continuous wearing by virtue of such non-interference. In some implementations, it can further be desirable that the subject may have no notion about when the sensing device(s) of the ambulatory monitoring device is actually performing measurements.

Moreover, some disclosed implementations provide advantages compared to previously-deployed non-invasive blood pressure monitoring devices, such as those based on photoplethysmography (PPG). PPG-based blood pressure monitoring devices are not optimal because PPG superimposes data corresponding to the blood volume of all illuminated blood vessels (arteries, veins, etc.), each of which exhibit unique blood volume changes over time, thereby producing a blended signal that is not closely correlated to blood pressure and is susceptible to drift. In contrast, some disclosed devices apply depth-discriminated photoacoustic plethysmography (PAPG) methods, which can distinguish artery heart rate waveforms from vein heart rate waveforms and other heart rate waveforms. Blood pressure estimation based on depth-discriminated PAPG methods can be substantially more accurate than blood pressure estimation based on PPG-based methods. Some disclosed methods have the additional potential advantage of applying more than one type of blood pressure estimation method that is based on depth-discriminated PAPG methods, thereby providing a potentially more reliable blood pressure estimation. Alternatively, or additionally, some disclosed methods have the additional potential advantage of providing one or more PAPG-based blood pressure estimation methods that are based on pulse transit time (PTT).

As used herein, the term "pulse pressure" refers to the difference between the systolic pressure and the diastolic pressure for a given cardiac cycle. Pulse pressure is generally not affected by local changes in the hydrostatic pressure in an artery in the peripheral regions of the body of a subject. As used herein, the term "transmural pressure" refers to the pressure difference between the pressure inside a particular artery and the pressure directly outside the artery at a particular time and at a particular location along the artery. Unlike the pulse pressure, the transmural pressure depends on hydrostatic pressure. For example, if a sensing device is coupled with a wrist of a subject, changing the elevation of the wrist can cause significant changes in the transmural pressure measured at the wrist, while the pulse pressure will generally be relatively unaffected (assuming the state of the subject is otherwise unchanged). As used herein, the term "absolute arterial pressure" refers to the actual pressure in a particular artery at a particular location along the artery at a particular time. Typically, the absolute arterial pressure is relatively consistent with the transmural pressure so long as no significant external pressure is applied to the artery (such as from a counter pressure applied by an inflatable cuff or other external device). For many intents and purposes, the transmural pressure may be presumed to be approximately equal to the absolute arterial pressure, and as such, the terms "absolute arterial pressure" and "transmural pressure" are used interchangeably hereinafter where appropriate unless otherwise noted. As used herein, the term "blood pressure" is a general term referring to a pressure in the arterial system of a subject. As such, the terms transmural pressure, absolute arterial pressure, pulse pressure, systolic pressure and diastolic pressure all may referred to hereinafter generally as blood pressure.

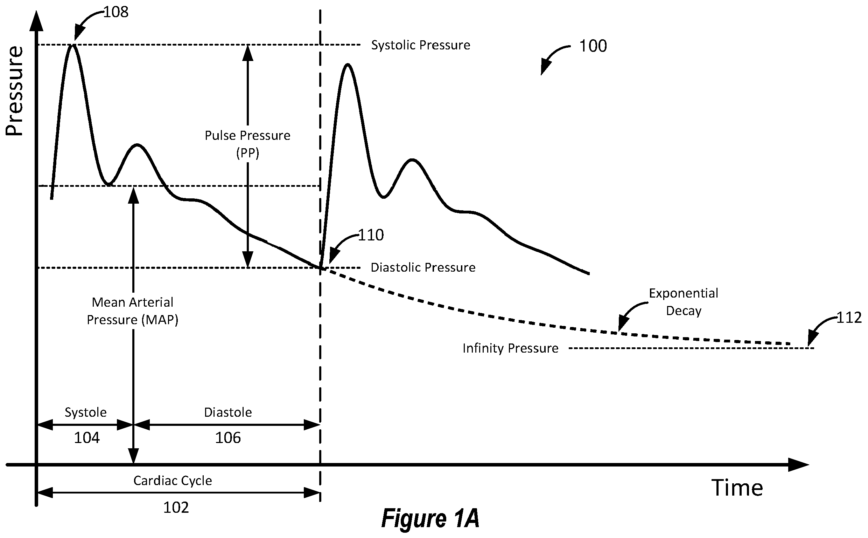

Figure 1A shows a plot 100 of a blood pressure signal in an example artery during an example cardiac cycle. Although the plot 100 is a plot of blood pressure versus time, the plot 100 also is indicative of the arterial distension waveform. As indicated above, a plot of blood flow versus time would exhibit similar features as the plot 100 of blood pressure versus time, although the specific shapes of the features would be slightly different. As a person of ordinary skill in the art will appreciate, each cardiac cycle 102 includes both a systolic phase ("ventricular systole") 104, during which the left ventricle of the heart contracts and pumps blood into the arterial system, and a diastolic phase ("ventricular diastole") 106, during which the left ventricle relaxes and fills with blood in preparation for the next systolic phase. Because each cardiac cycle 102 yields a respective pressure pulse, the arterial distension waveform associated with each pressure pulse also includes features characteristic of the systolic and diastolic phases. For example, the systolic phase 104 characteristically includes a rapid rise of the pressure culminating in a local maximum or peak 108 (the "systolic pressure") responsive to the injection of blood from the left ventricle during the given cardiac cycle 102. The diastolic phase 106, on the contrary, characteristically includes a marked drop in blood pressure culminating in a local minimum 110 (the "diastolic pressure") during the given cardiac cycle 102 as a consequence of the relaxation of the left ventricle. In fact, the ending portion of the diastolic phase 106 can generally be characterized by an exponentially decaying blood pressure that asymptotically approaches a pressure 112 (referred to herein as the "infinity pressure") lower than the typical diastolic pressure (the blood pressure never reaches the infinity pressure because the systolic phase of the next cardiac cycle interrupts the exponential decay as shown).

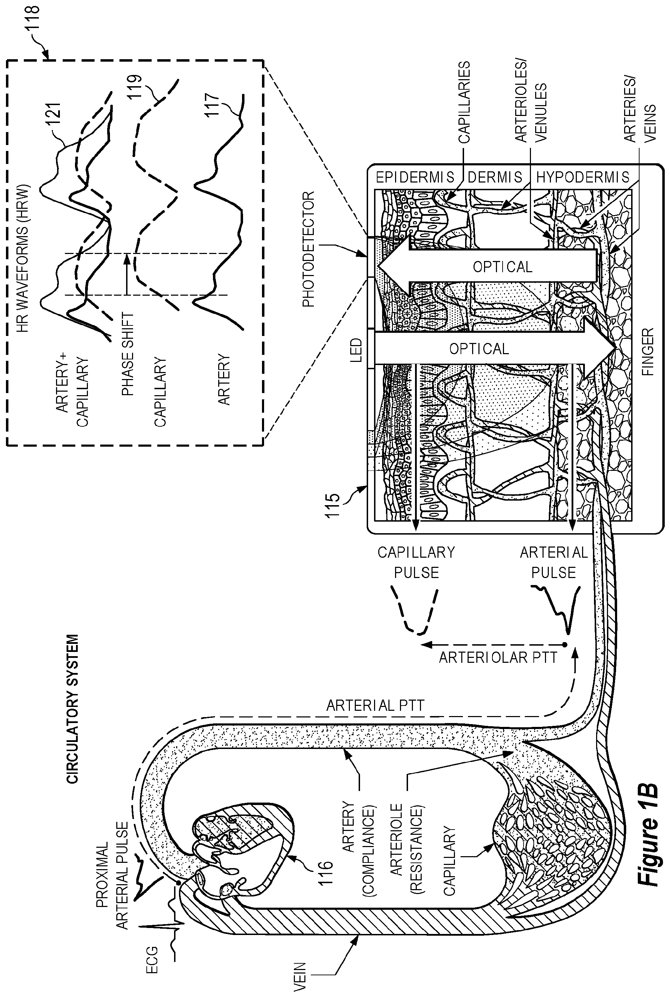

Figure 1B shows an example of a blood pressure monitoring device based on photoplethysmography (PPG). Figure 1B shows examples of arteries, veins, arterioles, venules and capillaries of a circulatory system, including those inside a finger 115. In the example shown in Figure 1B, an electrocardiogram sensor has detected a proximal arterial pulse near the heart 116. Some examples are described below of measurement of the arterial pulse transit time (PTT) according to arterial pulses measured by two sensors, one of which may be an electrocardiogram sensor in some implementations.

According to the example shown in Figure 1B, a light source that includes one or more light-emitting diodes (LEDs) has transmitted light (in some examples, green, red, and/or near-infrared (NIR) light) that has penetrated the tissues of the finger 115 in an illuminated zone. Reflections from these tissues, detected by the photodetector, may be used to detect volumetric changes in the blood of the illuminated zone of the finger 115 that correspond to heart rate waveforms.

As shown in the heart rate waveform graphs 118 of Figure 1B, the capillary heart rate waveform 119 is differently-shaped and phase-shifted relative to the artery heart rate waveform 117. In this simple example, the detected heart rate waveform 121 is a combination of the capillary heart rate waveform 119 and the artery heart rate waveform 117. In some instances, the responses of one or more other blood vessels may also be part of the heart rate waveform 121 detected by a PPG-based blood pressure monitoring device.

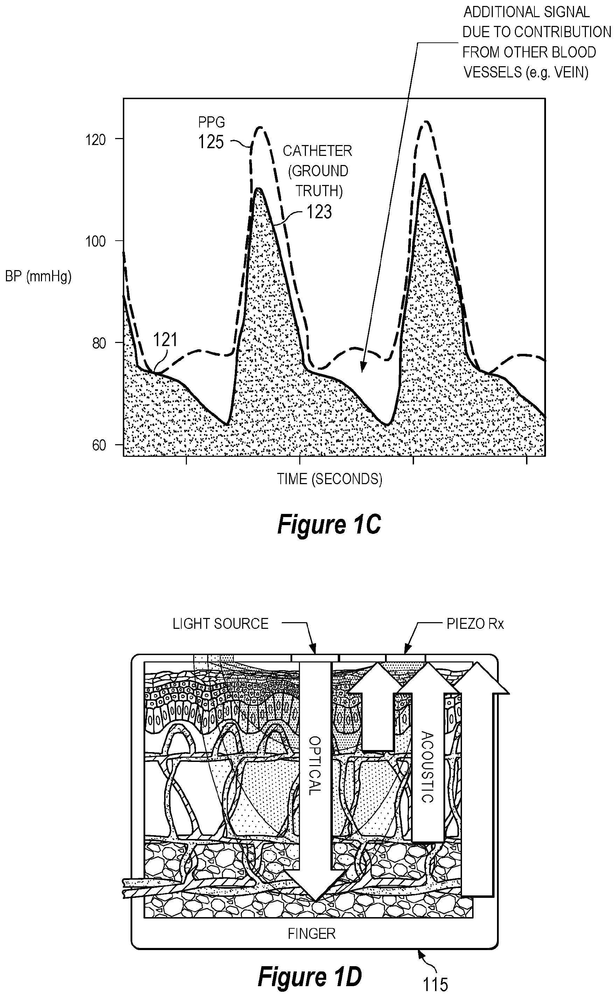

Figure 1C shows an example of two superimposed graphs of blood pressure variation during cardiac cycles. The graph 123 corresponds to blood pressure measured by a catheter, which is a sufficiently reliable method to be considered a "ground truth" against which blood pressure estimation methods can be compared. In this example, the graph 125 corresponds to blood pressure estimated by a PPG-based method. In the example shown in Figure 1C, the areas between the graph 123 and the graph 125 indicate the errors in blood pressure estimation according to the PPG-based method.

By comparing the heart rate waveform graphs 118 of Figure 1B and the blood pressure graphs of Figure 1C, one can appreciate that PPG-based blood pressure monitoring devices are not optimal because PPG superimposes data corresponding to the blood volume of all illuminated blood vessels, each of which exhibit different and time-shifted blood volume changes.

According to the example shown in Figure 1B, a light source that includes one or more LEDs has transmitted light (in some examples, green, red, and/or near-infrared (NIR) light) that has penetrated the tissues of the finger 115 in an illuminated zone. Reflections from these tissues, detected by the photodetector, may be used to detect volumetric changes in the blood of the illuminated zone of the finger 115 that correspond to heart rate waveforms.

Figure 1D shows an example of a blood pressure monitoring device based on photoacoustic plethysmography, which may be referred to herein as PAPG. Figure 1D shows the same examples of arteries, veins, arterioles, venules and capillaries inside the finger 115 that are shown in Figure 1B. In some examples, the light source shown in Figure 1D may be, or may include, one or more LEDs or laser diodes. In this example, as in Figure 1B, the light source has transmitted light (in some examples, green, red, and/or near-infrared (NIR) light) that has penetrated the tissues of the finger 115 in an illuminated zone.

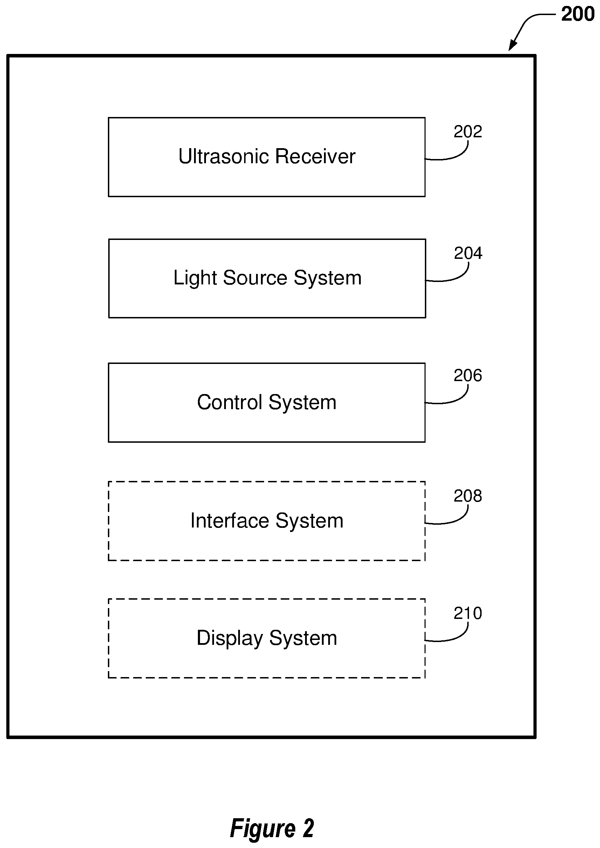

In the example shown in Figure 1D, blood vessels (and components of the blood itself) are heated by the incident light from the light source and are emitting acoustic waves. In this example, the emitted acoustic waves include ultrasonic waves. According to this implementation, the acoustic wave emissions are being detected by an ultrasonic receiver, which is a piezoelectric receiver in this example. Photoacoustic emissions from the illuminated tissues, detected by the piezoelectric receiver, may be used to detect volumetric changes in the blood of the illuminated zone of the finger 115 that correspond to heart rate waveforms. In some examples, the ultrasonic receiver may correspond to the ultrasonic receiver 202 that is described below with reference to Figure 2.

One important difference between the PPG-based system of Figure 1B and the PAPG-based method of Figure 1D is that the acoustic waves shown in Figure 1D travel much more slowly than the reflected light waves shown in Figure 1B. Accordingly, depth discrimination based on the arrival times of the acoustic waves shown in Figure 1D is possible, whereas depth discrimination based on the arrival times of the light waves shown in Figure 1B may not be possible. This depth discrimination allows some disclosed implementations to isolate acoustic waves received from the different blood vessels.

According to some such examples, such depth discrimination allows artery heart rate waveforms to be distinguished from vein heart rate waveforms and other heart rate waveforms. Therefore, blood pressure estimation based on depth-discriminated PAPG methods can be substantially more accurate than blood pressure estimation based on PPG-based methods. Some disclosed methods have the additional potential advantage of applying more than one type of blood pressure estimation method that is based on depth-discriminated PAPG methods, thereby providing a potentially yet more reliable blood pressure estimation.

Figure 2 is a block diagram that shows example components of an apparatus according to some disclosed implementations. In this example, the apparatus 200 includes a biometric system. Here, the biometric system includes an ultrasonic receiver 202, a light source system 204 and a control system 206. Although not shown in Figure 2, the apparatus 200 may include a substrate. In some examples, the apparatus 200 may include a platen. Some examples are described below. Some implementations of the apparatus 200 may include the interface system 208 and/or the display system 210.

Various examples of ultrasonic receivers 202 are disclosed herein, some of which may include, or be configured (or configurable) as, an ultrasonic transmitter and some of which may not. In some implementations the ultrasonic receiver 202 and an ultrasonic transmitter may be combined in an ultrasonic transceiver. In some examples, the ultrasonic receiver 202 may include a piezoelectric receiver layer, such as a layer of PVDF polymer or a layer of PVDF-TrFE copolymer. In some implementations, a single piezoelectric layer may serve as an ultrasonic receiver. In some implementations, other piezoelectric materials may be used in the piezoelectric layer, such as aluminum nitride (AlN) or lead zirconate titanate (PZT). The ultrasonic receiver 202 may, in some examples, include an array of ultrasonic transducer elements, such as an array of piezoelectric micromachined ultrasonic transducers (PMUTs), an array of capacitive micromachined ultrasonic transducers (CMUTs), etc. In some such examples, a piezoelectric receiver layer, PMUT elements in a single-layer array of PMUTs, or CMUT elements in a single-layer array of CMUTs, may be used as ultrasonic transmitters as well as ultrasonic receivers. According to some examples, the ultrasonic receiver 202 may be, or may include, an ultrasonic receiver array. In some examples, the apparatus 200 may include one or more separate ultrasonic transmitter elements. In some such examples, the ultrasonic transmitter(s) may include an ultrasonic plane-wave generator.

The light source system 204 may, in some examples, include an array of light-emitting diodes. In some implementations, the light source system 204 may include one or more laser diodes. According to some implementations, the light source system may include at least one infrared, red, green, blue, white or ultraviolet light-emitting diode. In some implementations, the light source system 204 may include one or more laser diodes. For example, the light source system 204 may include at least one infrared, red, green, blue, white or ultraviolet laser diode. In some implementations, the light source system 204 may include one or more organic LEDs (OLEDs).

In some implementations, the light source system 204 may be configured for emitting various wavelengths of light, which may be selectable in order to achieve greater penetration into biological tissue and/or to trigger acoustic wave emissions primarily from a particular type of material. For example, because near-infrared (near-IR) light is not as strongly absorbed by some types of biological tissue (such as melanin and blood vessel tissues) as relatively shorter wavelengths of light, in some implementations the light source system 204 may be configured for emitting one or more wavelengths of light in the near IR range, in order to obtain photoacoustic emissions from relatively deep biological tissues. In some such implementations the control system 206 may control the wavelength(s) of light emitted by the light source system 204 to be in the range of 750 to 850 nm, e.g., 808 nm. However, hemoglobin does not absorb near-IR light as much as hemoglobin absorbs light having shorter wavelengths, e.g., ultraviolet, violet, blue or green light. Near-IR light can produce suitable photoacoustic emissions from some blood vessels (e.g., 1 mm in diameter or larger), but not necessarily from very small blood vessels. In order to achieve greater photoacoustic emissions from blood in general and from smaller blood vessels in particular, in some implementations the control system 206 may control the wavelength(s) of light emitted by the light source system 204 to be in the range of 495 to 570 nm, e.g., 520 nm or 532 nm. Wavelengths of light in this range are more strongly absorbed by biological tissue and therefore may not penetrate the biological tissue as deeply, but can produce relatively stronger photoacoustic emissions in blood than near-IR light. In some examples the control system 206 may control the wavelength(s) of light emitted by the light source system 204 to preferentially induce acoustic waves in blood vessels, other soft tissue, and/or bones. For example, an infrared (IR) light-emitting diode LED may be selected and a short pulse of IR light emitted to illuminate a portion of a target object and generate acoustic wave emissions that are then detected by the ultrasonic receiver 202. In another example, an IR LED and a red LED or other color such as green, blue, white or ultraviolet (UV) may be selected and a short pulse of light emitted from each light source in turn with ultrasonic images obtained after light has been emitted from each light source. In other implementations, one or more light sources of different wavelengths may be fired in turn or simultaneously to generate acoustic emissions that may be detected by the ultrasonic receiver. Image data from the ultrasonic receiver that is obtained with light sources of different wavelengths and at different depths (e.g., as discussed in detail below) into the target object may be combined to determine the location and type of material in the target object. Image contrast may occur as materials in the body generally absorb light at different wavelengths differently. As materials in the body absorb light at a specific wavelength, they may heat differentially and generate acoustic wave emissions with sufficiently short pulses of light having sufficient intensities. Depth contrast may be obtained with light of different wavelengths and/or intensities at each selected wavelength. That is, successive images may be obtained at a fixed RGD (which may correspond with a fixed depth into the target object) with varying light intensities and wavelengths to detect materials and their locations within a target object. For example, hemoglobin, blood glucose and/or blood oxygen within a blood vessel inside a target object such as a finger may be detected photoacoustically.

According to some implementations, the light source system 204 may be configured for emitting a light pulse with a pulse width less than about 100 nanoseconds. In some implementations, the light pulse may have a pulse width between about 10 nanoseconds and about 500 nanoseconds or more. According to some examples, the light source system may be configured for emitting a plurality of light pulses at a pulse repetition frequency between 10 Hz and 100 kHz. Alternatively, or additionally, in some implementations the light source system 204 may be configured for emitting a plurality of light pulses at a pulse repetition frequency between about 1 MHz and about 100 MHz. Alternatively, or additionally, in some implementations the light source system 204 may be configured for emitting a plurality of light pulses at a pulse repetition frequency between about 10 Hz and about 1 MHz. In some examples, the pulse repetition frequency of the light pulses may correspond to an acoustic resonant frequency of the ultrasonic receiver and the substrate. For example, a set of four or more light pulses may be emitted from the light source system 204 at a frequency that corresponds with the resonant frequency of a resonant acoustic cavity in the sensor stack, allowing a build-up of the received ultrasonic waves and a higher resultant signal strength. In some implementations, filtered light or light sources with specific wavelengths for detecting selected materials may be included with the light source system 204. In some implementations, the light source system may contain light sources such as red, green and blue LEDs of a display that may be augmented with light sources of other wavelengths (such as IR and/or UV) and with light sources of higher optical power. For example, high-power laser diodes or electronic flash units (e.g., an LED or xenon flash unit) with or without filters may be used for short-term illumination of the target object.

The control system 206 may include one or more general purpose single- or multi-chip processors, digital signal processors (DSPs), application specific integrated circuits (ASICs), field programmable gate arrays (FPGAs) or other programmable logic devices, discrete gates or transistor logic, discrete hardware components, or combinations thereof. The control system 206 also may include (and/or be configured for communication with) one or more memory devices, such as one or more random access memory (RAM) devices, read-only memory (ROM) devices, etc. Accordingly, the apparatus 200 may have a memory system that includes one or more memory devices, though the memory system is not shown in Figure 2. The control system 206 may be configured for receiving and processing data from the ultrasonic receiver 202, e.g., as described below. If the apparatus 200 includes an ultrasonic transmitter, the control system 206 may be configured for controlling the ultrasonic transmitter. In some implementations, functionality of the control system 206 may be partitioned between one or more controllers or processors, such as a dedicated sensor controller and an applications processor of a mobile device.

Some implementations of the apparatus 200 may include the interface system 208. In some examples, the interface system 208 may include a wireless interface system. In some implementations, the interface system 208 may include a user interface system, one or more network interfaces, one or more interfaces between the control system 206 and a memory system and/or one or more interfaces between the control system 206 and one or more external device interfaces (e.g., ports or applications processors).

According to some examples, the apparatus 200 may include a display system 210 that includes one or more displays. For example, the display system 210 may include one or more LED displays, such as one or more organic LED (OLED) displays.

The apparatus 200 may be used in a variety of different contexts, many examples of which are disclosed herein. For example, in some implementations a mobile device may include the apparatus 200. In some implementations, a wearable device may include the apparatus 200. The wearable device may, for example, be a bracelet, an armband, a wristband, a ring, a headband, an earbud or a patch.

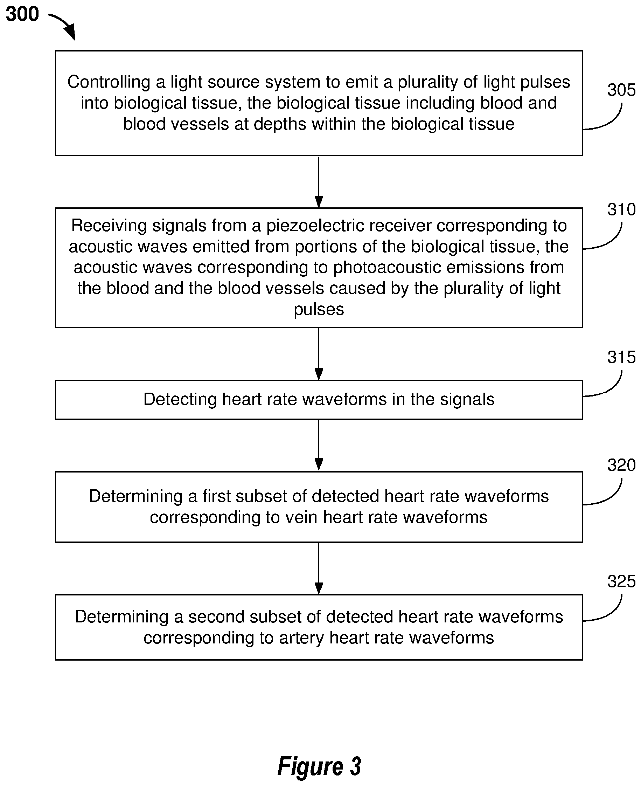

Figure 3 is a flow diagram that provides examples of some disclosed operations. The blocks of Figure 3 (and those of other flow diagrams provided herein) may, for example, be performed by the apparatus 200 of Figure 2 or by a similar apparatus. As with other methods disclosed herein, the method outlined in Figure 3 may include more or fewer blocks than indicated. Moreover, the blocks of methods disclosed herein are not necessarily performed in the order indicated. In some instances, one or more of the blocks shown in Figure 3 may be performed concurrently.

Here, block 305 involves controlling a light source system to emit light. In some such implementations, the control system 206 of the apparatus 200 may control the light source system 204 to emit light. According to this implementation, block 305 involves controlling the light source system to emit a plurality of light pulses into biological tissue including blood and blood vessels at depths within the biological tissue. In some such examples, block 305 involves controlling the light source system to emit a plurality of light pulses at a pulse repetition frequency. In some examples, the pulse repetition frequency may be in a range between, or including, 10Hz and 1 MHz.

In some implementations, the control system may be configured for selecting one or more wavelengths of light for the plurality of light pulses, e.g., as described above. According to some examples, the control system may be configured for selecting a light intensity associated with one or more selected wavelengths. For example, the control system may be configured for selecting one or more wavelengths of light and light intensities associated with each selected wavelength to generate acoustic wave emissions from one or more portions of the target object. In some examples, the control system may be configured for selecting the one or more wavelengths of light to evaluate a one or more characteristics of the target object, e.g., to evaluate blood oxygen levels. In some examples, block 305 may involve controlling a light source system to emit light that is transmitted through a substrate and/or other layers of an apparatus such as the apparatus 200.

According to this implementation, block 310 involves receiving signals from an ultrasonic receiver corresponding to acoustic waves emitted from portions of the biological tissue in response to being illuminated with light emitted by the light source system. In this implementation, the acoustic waves correspond to photoacoustic emissions from the blood and/or the blood vessels of the biological tissue caused by the plurality of light pulses. In this example, the ultrasonic receiver is, or includes, a piezoelectric receiver. In some instances a target object (such as a digit, a wrist or another body part) that includes the biological tissue may be positioned on a surface of the ultrasonic receiver or positioned on a surface of a device that includes the ultrasonic receiver. The ultrasonic receiver may, in some implementations, be the ultrasonic receiver 202 that is shown in Figure 2 and described above. In some examples, one or more coatings or acoustic matching layers (e.g., for matching the acoustic impedance of human skin) may reside on a surface of the ultrasonic receiver or a surface of a device that includes the ultrasonic receiver (e.g., a surface of a cover glass or a platen of the device).

In this example, block 315 involves detecting heart rate waveforms in the signals received from the ultrasonic receiver. According to this implementation, block 320 involves determining a first subset of detected heart rate waveforms corresponding to vein heart rate waveforms. In this example, block 325 involves determining a second subset of detected heart rate waveforms corresponding to artery heart rate waveforms. Various detailed examples of blocks 315, 320 and 325 are disclosed herein.

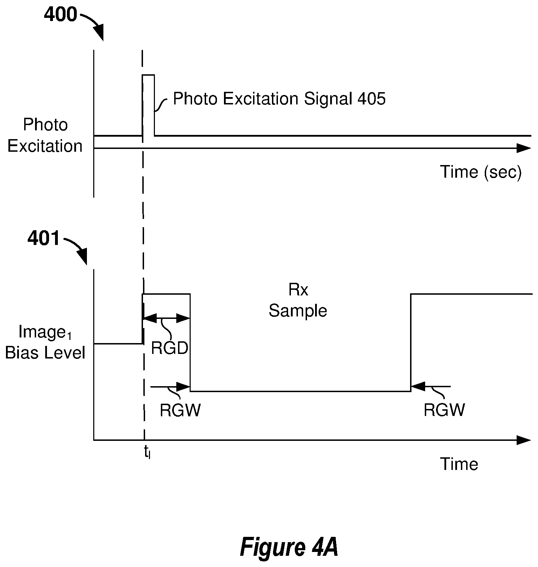

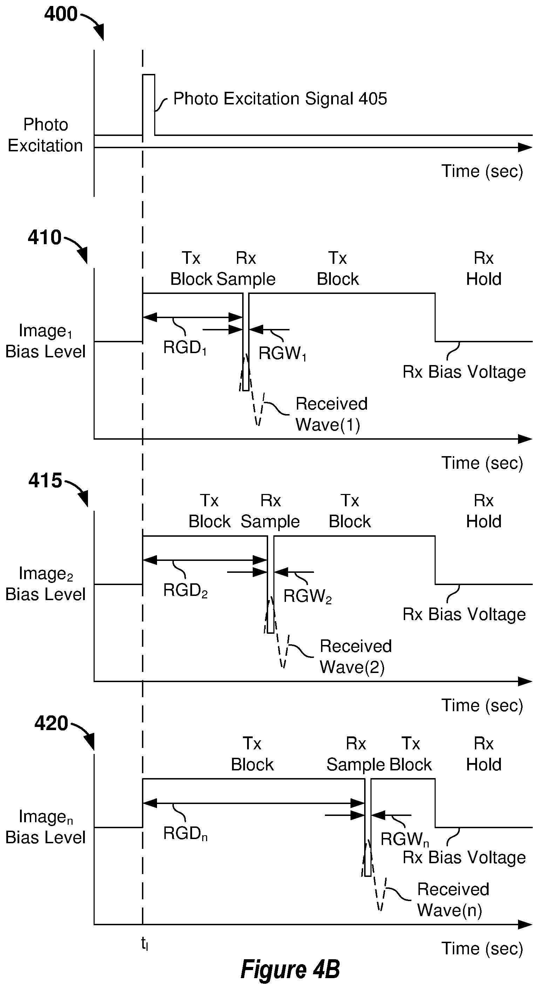

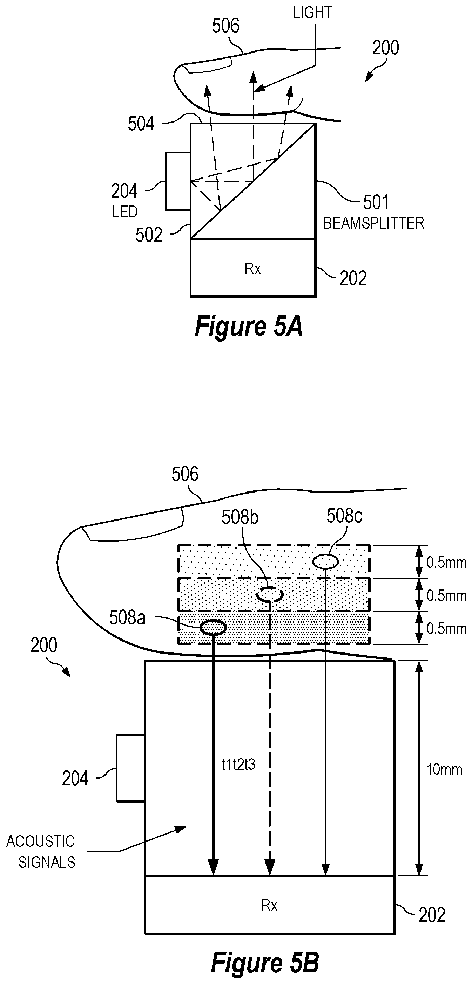

According to some examples, the control system may be configured for discriminating between vein heart rate waveforms and artery heart rate waveforms by obtaining depth-discriminated signals. Figure 4A shows an example of a range-gate window (RGW) selected to receive acoustic waves emitted from a range of different depths. The acquisition time delay or range gate delay (which is labeled "RGD" in Figure 4B) is measured from the beginning time t₁ of the photo-excitation signal 405 shown in graph 400. The RGD may, for example, be selected to correspond with the time required for photoacoustic emissions from a shallowest target of interest to reach a receiver, e.g., as described below with reference to Figures 5A and 5B. Accordingly, the RGD may depend on the particular arrangement of the apparatus being used to receive the photoacoustic emissions, including the thickness of the layer(s) between the target object and the receiver and the speed of sound of the layer(s) between the target object and the receiver. The graph 401 depicts a time after RGD during which emitted acoustic waves may be received and sampled by an ultrasonic receiver during an acquisition time window (also known as a range-gate window or a range-gate width) of RGW. In some implementations, the RGW may be 10 microseconds. Other implementations may have larger or smaller RGWs.

In some examples, depth-discriminated signals may be obtained by a process of partitioning the acoustic waves received during the RGW into a plurality of smaller time windows. Each of the time windows may correspond to a depth range inside the target object from which the acoustic waves are received. In some examples, the depth range or thickness of each layer may be 0.5 mm. Assuming a speed of sound of 1.5 mm/microsecond, each 0.5 mm layer would correspond to a time slot of approximately 0.33 microseconds. However, the depth range may vary according to the particular implementation.

According to some alternative examples, receiving the signals from the piezoelectric receiver involves obtaining depth-discriminated signals by applying first through *N^{th}* acquisition time delays and receiving first through *N^{th}* signals during first through *N^{th}* acquisition time windows, each of the first through *N^{th}* acquisition time windows occurring after a corresponding one of the first through *N^{th}* acquisition time delays, wherein *N* is an integer greater than one. The control system may be configured for determining the first subset of detected heart rate waveforms and the second subset of detected heart rate waveforms based, at least in part, on the depth-discriminated signals.

Figure 4B shows examples of multiple acquisition time delays being selected to receive acoustic waves emitted from different depths. In these examples, each of the acquisition time delays (which are labeled range-gate delays or RGDs in Figure 4B) is measured from the beginning time t₁ of the photo-excitation signal 405 shown in graph 400. The graph 410 depicts emitted acoustic waves (received wave (1) is one example) that may be received by an ultrasonic sensor array at an acquisition time delay RGD₁ and sampled during an acquisition time window (also known as a range-gate window or a range-gate width) of RGW₁. Such acoustic waves will generally be emitted from a relatively shallower portion of a target object proximate, or positioned upon, a platen of the biometric system.

Graph 415 depicts emitted acoustic waves (received wave (2) is one example) that are received by the ultrasonic sensor array at an acquisition time delay RGD₂ (with RGD₂ > RGD₁) and sampled during an acquisition time window of RGW₂. Such acoustic waves will generally be emitted from a relatively deeper portion of the target object.

Graph 420 depicts emitted acoustic waves (received wave (n) is one example) that are received at an acquisition time delay RGDₙ (with RGDₙ > RGD₂ > RGD₁) and sampled during an acquisition time window of RGWₙ. Such acoustic waves will generally be emitted from a still deeper portion of the target object. Range-gate delays are typically integer multiples of a clock period. A clock frequency of 128 MHz, for example, has a clock period of 7.8125 nanoseconds, and RGDs may range from under 10 nanoseconds to over 2000 nanoseconds. Similarly, the range-gate widths may also be integer multiples of the clock period, but are often much shorter than the RGD (e.g. less than about 50 nanoseconds) to capture returning signals while retaining good axial resolution. In some implementations, the acquisition time window (e.g. RGW) may be between 175 nanoseconds to 320 nanoseconds or more. In some examples, the RGW may be more or fewer nanoseconds, e.g., in the range of 25 nanoseconds to 1000 nanoseconds.

Figures 5A and 5B shows examples of an apparatus configured to receive acoustic waves emitted from different depths. The apparatus shown in Figures 5A and 5B is an example of the apparatus 200 that is shown in Figure 2. As with the other implementations shown and described herein, the types of elements, the arrangement of the elements and the dimensions of the elements illustrated in Figures 5A and 5B are merely shown by way of example.

According to this example, the apparatus 200 includes an ultrasonic receiver 202, a light source system 204 (which includes an LED in this example) and a control system (which is not shown in Figures 5A and 5B). According to this implementation, the apparatus 200 includes a beamsplitter 501 onto a side 502 to which the LED is mounted. In this instance, a finger 506 rests upon an adjacent side 504 of the beamsplitter 501.

Figure 5A shows light emitted from the light source system 204, part of which is reflected by the beamsplitter 501 and enters the finger 506. The range gate delay for this implementation and other implementations may, for example, be selected to correspond with the time required for photoacoustic emissions from a shallowest target of interest to reach a receiver. For example, in one configuration of the apparatus 200 which uses a 12.7mm beamsplitter between the finger 506 and the ultrasonic receiver 202 (RX in Figure 5A), the finger surface signal will arrive at the time it takes the acoustic waves to travel through the entire beamsplitter. Using the speed of sound of borosilicate glass of 5500m/s as an approximate speed of sound for the beamsplitter and with the beamsplitter size of 12.7mm, this time becomes 12.7mm / 5500m/s or 2.3us. Therefore, a range gate delay of 2.3µs corresponds to the surface of the finger 506. To travel 1mm into the finger 506, for example, using the speed of sound for tissue now of 1. 5mm/us, this time becomes 1mm/1.5mm/µs or ~0.67µs. Therefore, a range gate delay of ~2.97µs (2.3µs + 0.67µs) would cause the ultrasonic receiver 202 to begin sampling acoustic waves reflected from a depth of approximately 1mm below the outer surface of the finger 506.

Figure 5B shows acoustic signals corresponding to photoacoustic emissions from tissues (e.g., blood and blood vessels) inside the finger 506, caused by the light that entered the finger 506. In the example shown in Figure 5B, the acoustic signals originate from different depths (depths 508a, 508b and 508c) within the finger 506. Accordingly, the travel times t1, t2 and t3, from the depths 508a, 508b and 508c, respectively, to the ultrasonic receiver 202, are also different: in this instance, t3 > t2 > t1. Therefore, multiple acquisition time delays may be selected to receive acoustic waves emitted from the depths 508a, 508b and 508c, e.g., as shown in Figure 4B and described above.

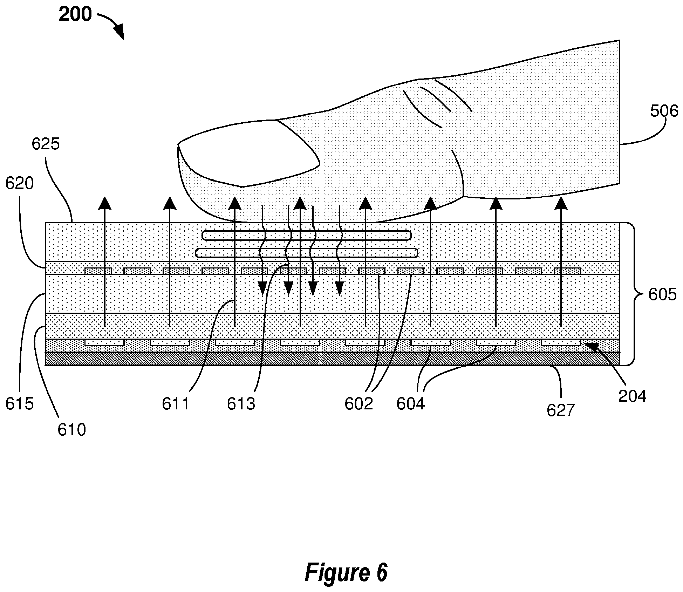

Figure 6 shows an example of a cross-sectional view of an apparatus capable of performing the method of Figure 3. The apparatus 200 shown in Figure 6 is another example of the apparatus 200 that is described above with reference to Figure 2. As with the other implementations shown and described herein, the types of elements, the arrangement of the elements and the dimensions of the elements illustrated in Figure 6 are merely shown by way of example.

Figure 6 shows an example of a target object (the finger 506, in this instance) being illuminated by incident light and subsequently emitting acoustic waves. In this example, the apparatus 200 includes a light source system 204, which may include an array of light-emitting diodes and/or an array of laser diodes. In some implementations, the light source system 204 may be capable of emitting various wavelengths of light, which may be selectable to trigger acoustic wave emissions primarily from a particular type of material. In some instances, the incident light wavelength, wavelengths and/or wavelength range(s) may be selected to trigger acoustic wave emissions primarily from a particular type of material, such as blood, blood vessels, other soft tissue, or bones. To achieve sufficient image contrast, light sources 604 of the light source system 204 may need to have a higher intensity and optical power output than light sources generally used to illuminate displays. In some implementations, light sources with light output of 1-100 millijoules or more per pulse, e.g., 10 millijoules per pulse, with pulse widths in the range of 100 nanoseconds to 600 nanoseconds, may be suitable. In some implementations, the pulse width of the emitted light may be between 10 nanoseconds and 700 nanoseconds.

In this example, incident light 611 has been transmitted from the light sources 604 of the light system 204 through a sensor stack 605 and into an overlying finger 506. The various layers of the sensor stack 605 may include one or more substrates of glass or other material such as plastic or sapphire that is substantially transparent to the light emitted by the light source system 204. In this example, the sensor stack 605 includes a substrate 610 to which the light source system 204 is coupled, which may be a backlight of a display according to some implementations. In alternative implementations, the light source system 204 may be coupled to a front light. Accordingly, in some implementations the light source system 204 may be configured for illuminating a display and the target object.

In this implementation, the substrate 610 is coupled to a thin-film transistor (TFT) substrate 615 for the ultrasonic receiver 202, which includes an array of sensor pixels 602 in this example. According to this example, a piezoelectric receiver layer 620 overlies the sensor pixels 602 of the ultrasonic receiver 202 and a platen 625 overlies the piezoelectric receiver layer 620. Accordingly, in this example the apparatus 200 is capable of transmitting the incident light 611 through one or more substrates of the sensor stack 605 that include the ultrasonic receiver 202 with substrate 615 and the platen 625 that may also be viewed as a substrate. In some implementations, sensor pixels 602 of the ultrasonic receiver 202 may be transparent, partially transparent or substantially transparent, such that the apparatus 200 may be capable of transmitting the incident light 611 through elements of the ultrasonic receiver 202. In some implementations, the ultrasonic receiver 202 and associated circuitry may be formed on or in a glass, plastic or silicon substrate.

According to some implementations, the apparatus 200 may include an ultrasonic transmitter 627, such as the ultrasonic transmitter 627 that is shown in Figure 6. The ultrasonic transmitter may or may not be part of the ultrasonic receiver 202, depending on the particular implementation. In some examples, the ultrasonic receiver 202 may include PMUT or CMUT elements that are capable of transmitting and receiving ultrasonic waves, and the piezoelectric receiver layer 620 may be replaced with an acoustic coupling layer. In some examples, the ultrasonic receiver 202 may include an array of pixel input electrodes and sensor pixels formed in part from TFT circuitry, an overlying piezoelectric receiver layer 620 of piezoelectric material such as PVDF or PVDF-TrFE, and an upper electrode layer positioned on the piezoelectric receiver layer sometimes referred to as a receiver bias electrode. In the example shown in Figure 6, at least a portion of the apparatus 200 includes an ultrasonic transmitter 627 that can function as a plane-wave ultrasonic transmitter. The ultrasonic transmitter 627 may, for example, include a piezoelectric transmitter layer with transmitter excitation electrodes disposed on each side of the piezoelectric transmitter layer.

Here, the incident light 611 causes optical excitation within the finger 506 and resultant acoustic wave generation. In this example, the generated acoustic waves 613 include ultrasonic waves. Acoustic emissions generated by the absorption of incident light may be detected by the ultrasonic receiver 202. A high signal-to-noise ratio may be obtained because the resulting ultrasonic waves are caused by optical stimulation instead of by reflection of transmitted ultrasonic waves.

In this example, the apparatus 200 includes a control system, although the control system is not shown in Figure 6. According to some examples, the control system may be configured for discriminating between vein heart rate waveforms and artery heart rate waveforms by obtaining depth-discriminated signals. According to some such examples, receiving the signals from the piezoelectric receiver involves obtaining depth-discriminated signals by selecting an acquisition time window to receive acoustic waves emitted from a range of different depths within a target object, such as a finger, a wrist, an ear, etc. In some examples, depth-discriminated signals may be obtained by a process of partitioning the acoustic waves received during the RGW into a plurality of smaller time windows, e.g., as described above. Each of the time windows may correspond to a depth range inside the target object from which the acoustic waves are received. According to some alternative examples, receiving the signals from the piezoelectric receiver involves obtaining depth-discriminated signals by applying first through *N^{th}* acquisition time delays and receiving first through *N^{th}* signals during first through *N^{th}* acquisition time windows, each of the first through *N^{th}* acquisition time windows occurring after a corresponding one of the first through *N^{th}* acquisition time delays, wherein *N* is an integer greater than one. The control system may be configured for determining vein heart rate waveforms and artery heart rate waveforms (the "first subset of detected heart rate waveforms" and the "second subset of detected heart rate waveforms" of Figure 3) based, at least in part, on the depth-discriminated signals.

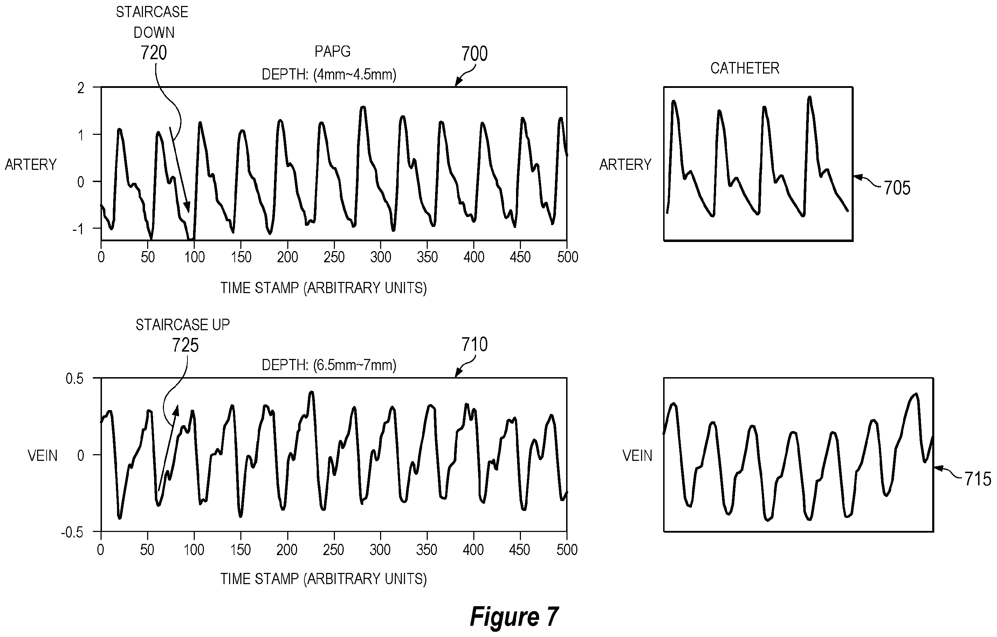

Figure 7 shows examples of vein heart rate waveforms and artery heart rate waveforms. In Figure 7, graph 700 shows examples of artery heart rate waveforms obtained using PAPG techniques like those described above with reference to Figures 3-6. In this example, depth-discriminated artery heart rate waveforms have been obtained from a depth of between 4.0 mm and 4.5 mm inside a finger.

In Figure 7, graph 710 shows examples of vein heart rate waveforms obtained using PAPG techniques such as those disclosed herein. According to this example, depth-discriminated vein heart rate waveforms have been obtained from a depth of between 6.5 mm and 7.0 mm inside the same finger. In the examples shown in graphs 700 and 710, the depth-discriminated PAPG waveforms are not cross-contaminated with waveforms from other depths, as would be the case if the waveforms had been obtained using PPG techniques.

Graph 705 shows examples of artery heart rate waveforms obtained via a catheter. Graph 715 shows examples of vein heart rate waveforms obtained via a catheter. As discussed with reference to Figure 1C, heart rate waveforms obtained via a catheter are known to be highly reliable and are considered by the present inventors to be a "ground truth" against which other techniques for obtaining heart rate waveforms may be compared. One may see that the "ground truth" artery heart rate waveforms of graph 705 strongly resemble the artery heart rate waveforms of graph 700, which were obtained using PAPG techniques. Likewise, one may see that the "ground truth" vein heart rate waveforms of graph 715 strongly resemble the vein heart rate waveforms of graph 710, which were obtained using PAPG techniques. This is strong evidence of the viability of the disclosed PAPG methods.

By referring to graphs 700 and 705, it may be observed that artery heart rate waveforms repeat a "staircase down" pattern, as shown by the arrow 720. By referring to graphs 710 and 715, it may be observed that vein heart rate waveforms repeat a "staircase up" pattern, as shown by the arrow 725.

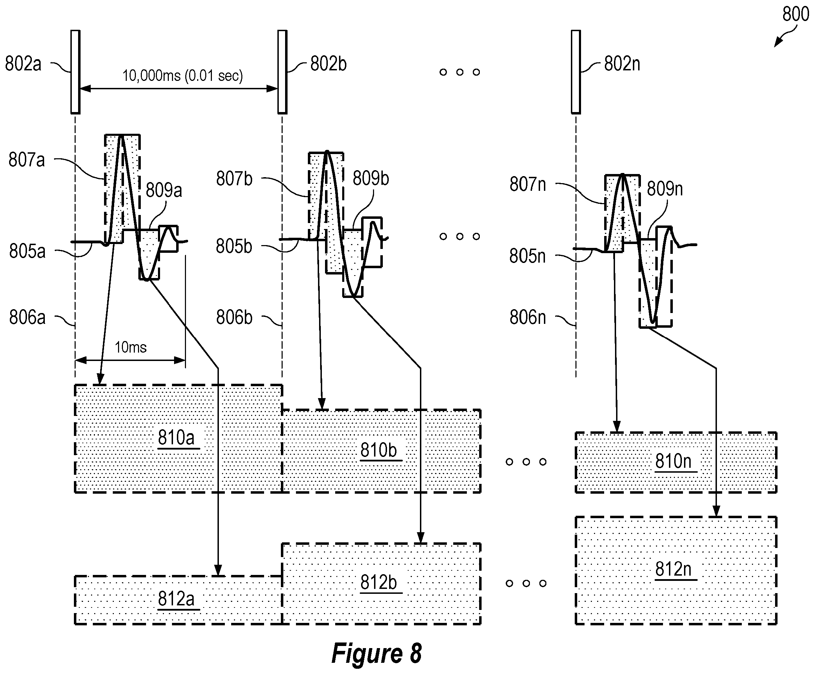

Figure 8 shows examples of determining vein heart rate waveforms and artery heart rate waveforms. Figure 8 depicts the signals 805a, 805b and 805n, which correspond to acoustic waves caused by photoacoustic emissions from biological tissue that includes blood and blood vessels. According to this example, there were additional signals 805c, 805d, etc., which are not shown in Figure 8.

The photoacoustic emissions corresponding to the signals 805a, 805b and 805n were caused by a plurality of corresponding light pulses 802a, 802b and 802n. In this example, there were additional light pulses 802c, 802d, etc., which are not shown in Figure 8. In this example, the light pulses 802a, 802b and 802n were separated in time by 10,000 microseconds or 0.01 seconds, corresponding to a pulse repetition frequency of 100 Hz. Other examples may involve other pulse repetition frequencies that are in a range between 10 Hz and 100 kHz, e.g., in a range between 50 Hz and 1000 Hz. According to some examples, the light pulses 802a, 802b and 802n may have a duration that is in the range of 2 nanoseconds to 5 microseconds. In some examples for which the light source system 204 includes one or more laser diodes, the light pulses 802a, 802b and 802n may have a duration that is in the range of 50 nanoseconds to 500 nanoseconds. In some examples for which the light source system 204 includes one or more lasers, the light pulses 802a, 802b and 802n may have a duration that is in the range of 5 nanoseconds to 100 nanoseconds.

In this example, the signals 805a, 805b and 805n were all received within 10 microseconds of the times at which the corresponding light pulses 802a, 802b and 802n were emitted, as suggested by the dashed lines 806a, 806b and 806n. However, the time scale used to represent the signals 805a, 805b and 805n is different from that used to represent the 10,000 microsecond time intervals between the light pulses 802a, 802b and 802n. In this example, the signals 805a, 805b and 805n were all received within a time interval corresponding to a single heart rate waveform.

The rectangles 807a and 809a represent samples of the received acoustic waves after the time of pulse 802a during RGWs of the same time duration, but after RGDs of different time durations. The RGD corresponding to the rectangle 807a is smaller than the RGD corresponding to the rectangle 809a. The RGD corresponding to the rectangle 807a was selected to receive acoustic waves generated by photoacoustic emissions from biological tissue at depths between approximately 2.0 and 2.5 mm. The RGD corresponding to the rectangle 809a was selected to receive acoustic waves generated by photoacoustic emissions from biological tissue at depths between approximately 3.0 and 3.5 mm.

The height of each of the rectangles 807a and 809a represents the absolute values of the difference between the maximum and minimum signal amplitudes (which may be referred to herein as "peak-to-peak values" or "peak-to-peak signal values") received during the corresponding time intervals. The rectangles 807b and 807n represent samples of received acoustic waves after the times of pulses 802b and 802n, during the same RGWs and after RGDs of the same duration as those for the rectangle 807a. The rectangles 809b and 809n represent samples of received acoustic waves after the times of pulses 802b and 802n, during the same RGWs and after RGDs of the same duration as those for the rectangle 809a. The heights of the rectangles 807b, 807n, and 809b and 809n represent the peak-to-peak values received during the corresponding time intervals.

The rectangle 810a corresponds to the peak-to-peak value of the rectangle 807a. Similarly, the rectangles 810b and 810n correspond to the peak-to-peak values of the rectangles 807b and 807n. It may be observed that the heights of the rectangles 810a, 810b and 810n are decreasing with time. This corresponds with the "staircase down" effect noted with respect to the artery waveforms of graphs 700 and 705 of Figure 7. Therefore, one may conclude that the rectangles 807a, 807b and 807n correspond to samples of waveforms received from an artery.

The rectangle 812a corresponds to the peak-to-peak value of the rectangle 809a. Similarly, the rectangles 812b and 812n correspond to the peak-to-peak values of the rectangles 809b and 809n. It may be observed that the heights of the rectangles 812a, 812b and 812n are increasing with time. This corresponds with the "staircase up" effect noted with respect to the vein waveforms of graphs 710 and 715 of Figure 7. Therefore, one may conclude that the rectangles 809a, 809b and 809n correspond to samples of waveforms received from a vein.

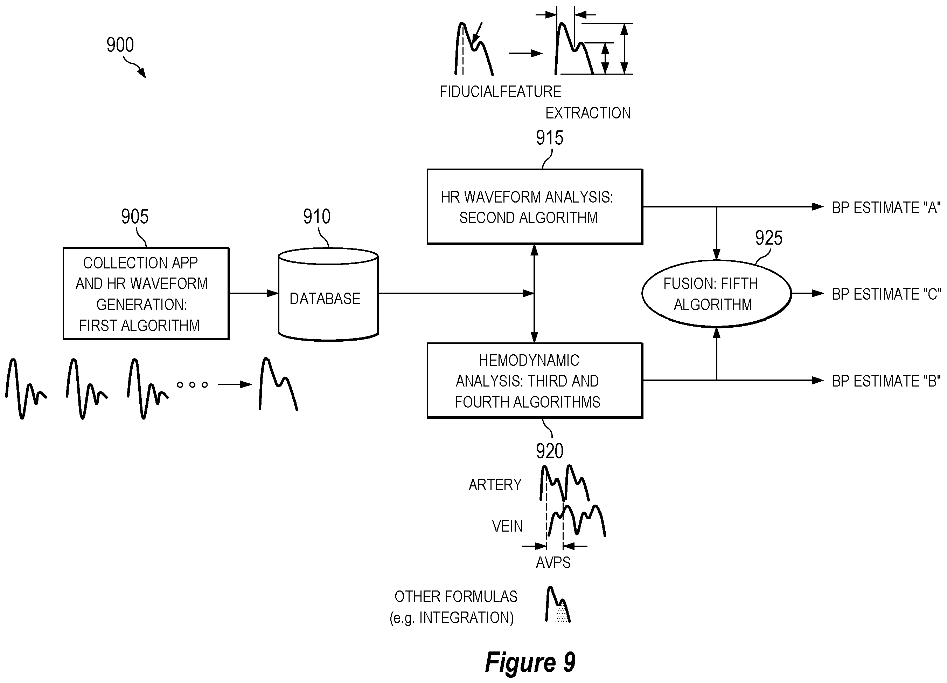

Figure 9 is a diagram that represents aspects of five disclosed PAPG algorithms. As used herein, the term "algorithm" refers to a method or to a set of two or more methods. An "algorithm" may or may not correspond to a particular mathematical formula or to a particular sequence of mathematical formulae, depending on the particular implementation. The blocks of Figure 9 (and those of other diagrams provided herein) may, for example, be performed by the apparatus 200 of Figure 2 or by a similar apparatus. As with other methods disclosed herein, the methods outlined in Figure 9 may include more or fewer blocks than indicated. Moreover, the blocks of methods disclosed herein are not necessarily performed in the order indicated. In some instances, one or more of the blocks shown in Figure 9 may be performed concurrently.

In this example, block 905 involves obtaining PAPG data and detecting heart rate waveforms. According to some examples, block 905 may involve performing one or more blocks of the method that is described above with reference to Figure 3. In some implementations, block 905 may involve obtaining depth-discriminated signals by performing one or more of the methods disclosed herein.

Block 905 may, in some instances, involve controlling a light source system to emit a plurality of light pulses into biological tissue at the pulse repetition frequency, the biological tissue including blood and blood vessels at depths within the biological tissue. Block 905 may involve receiving signals from a piezoelectric receiver corresponding to acoustic waves emitted from portions of the biological tissue. The acoustic waves may correspond to photoacoustic emissions from the blood and the blood vessels caused by the plurality of light pulses. Block 905 may involve detecting heart rate waveforms in the signals.

In some examples, block 905 may involve obtaining depth-discriminated signals by applying first through *N^{th}* acquisition time delays and receiving first through *N^{th}* signals during first through *N^{th}* acquisition time windows, each of the first through *N^{th}* acquisition time windows occurring after a corresponding one of the first through *N^{th}* acquisition time delays, wherein *N* is an integer greater than one. Block 905 may involve determining a first subset of detected heart rate waveforms corresponding to vein heart rate waveforms and a second subset of detected heart rate waveforms corresponding to artery heart rate waveforms based, at least in part, on the depth-discriminated signals.

In some implementations, block 905 may involve obtaining PAPG data and detecting heart rate waveforms from different elevations relative to a user's heart. For example, block 905 may involve obtaining a first set of PAPG data from a user's digit or wrist while the digit or wrist is at approximately the same elevation as a user's heart, obtaining a second set of PAPG data from the user's digit or wrist while the digit or wrist is at an elevation above the user's heart (e.g., with the user's arm extended above the user's head when the user is standing or seated in an upright position) and obtaining a third set of PAPG data from the user's digit or wrist while the digit or wrist is at an elevation below the user's heart (e.g., with the user's arm extended downward when the user is standing or seated in an upright position).

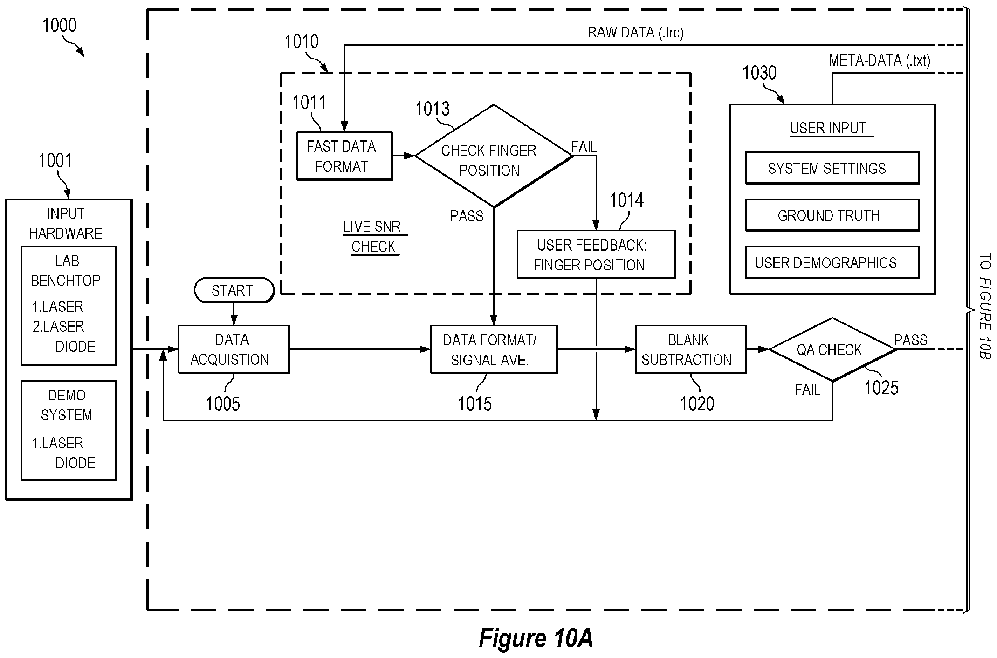

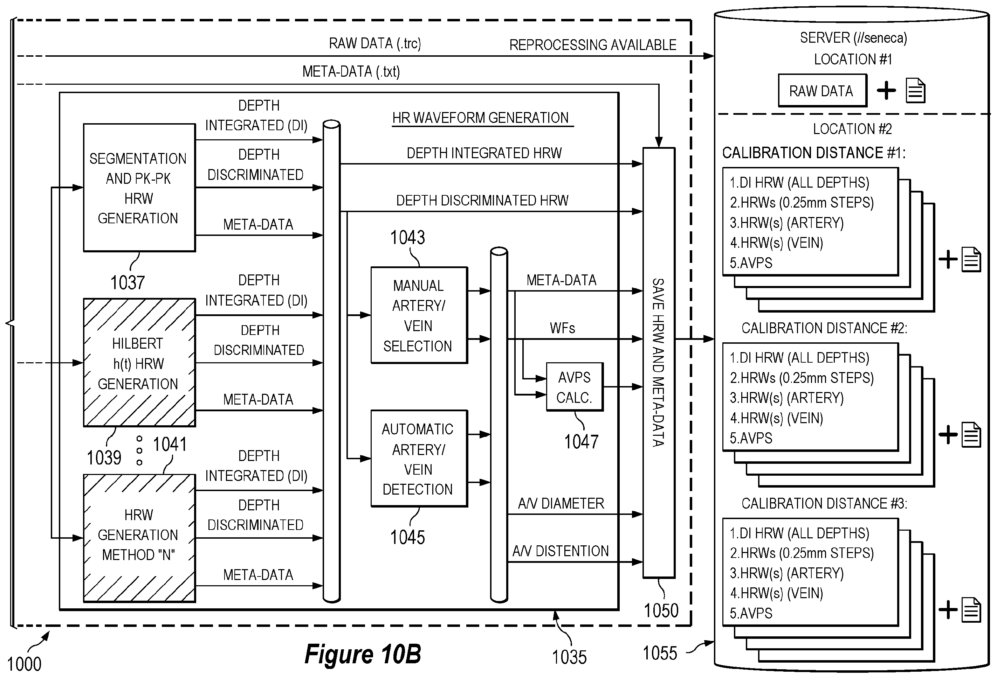

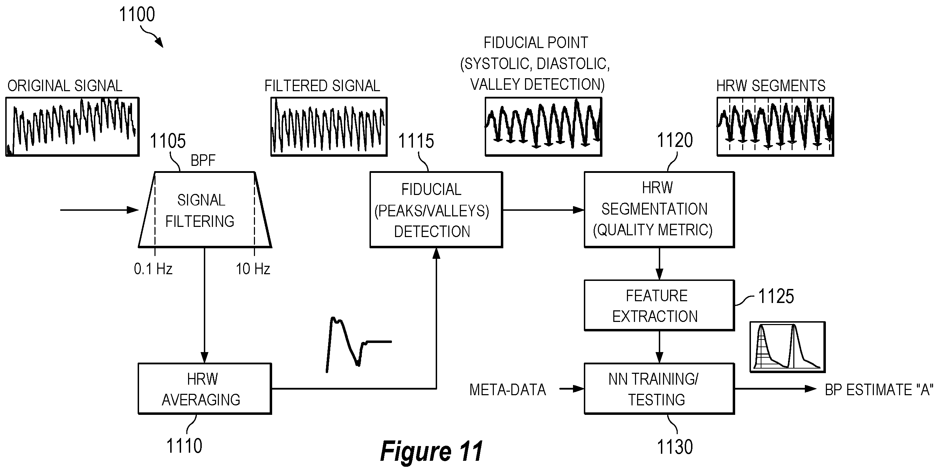

According to some examples, block 905 may involve at least some of the procedures that are described above with reference to Figure 8 or described below with reference to Figures 10A and 10B or Figure 11. In this example, the results of block 905 are stored in a database in block 910. According to the example shown in Figure 9, at least some of the results of block 905 are available for the processes of blocks 915 and 920, and in some instances for the processes of block 925.

In the example shown in Figure 9, block 915 involves extracting heart rate waveform features from the heart rate waveforms and making a blood pressure estimation based, at least in part, on extracted heart rate waveform features. Block 915 may involve heart rate waveform segmentation and the detection of systolic and diastolic portions of heart rate waveforms. Block 915 may, for example, involve peak and valley detection, which may be referred to herein as "fiducial point" detection. The term "heart rate waveform features," as used herein, includes such detected fiducial points. According to some examples, block 915 also may involve the detection of various types of heart rate waveform features corresponding to the widths of various portions of the heart rate waveforms, and in some instances metrics that are based on various combinations of such width values. Some detailed examples are described below with reference to Figure 11.

According to this example, block 920 involves making at least one blood pressure estimation based on hemodynamic analyses. In some examples, block 920 may involve extracting a set of hemodynamic features from the second subset of heart rate waveforms corresponding to artery heart rate waveforms and making a first blood pressure estimation based, at least in part, on the set of hemodynamic features. According to some implementations, block 920 may involve determining artery-vein phase shift (AVPS) data from the first subset of heart rate waveforms and the second subset of heart rate waveforms and making the first blood pressure estimation based, at least in part, on the AVPS data. Alternatively, or additionally, in some examples a blood pressure estimation may be based on the AVPS data alone. According to some examples, block 920 may involve making the first blood pressure estimation based, at least in part, on determining the area under one or more portions of a curve defined by an artery heart rate waveform. Some detailed examples are described below.

According to some examples, block 925 involves making a blood pressure estimate based on a combination of methods of blocks 915 and 920. Some such examples may involve making a first blood pressure estimation based, at least in part, on a set of hemodynamic features (block 920), making a second blood pressure estimation based, at least in part, on extracted heart rate waveform features (block 915) and making a third blood pressure estimation based, at least in part, on the first blood pressure estimation and the second blood pressure estimation. However, in some examples block 925 may involve making a first blood pressure estimation based, at least in part, on the AVPS data (block 920), making a second blood pressure estimation based, at least in part, on extracted heart rate waveform features (block 915) and making a third blood pressure estimation based, at least in part, on the first blood pressure estimation and the second blood pressure estimation.

According to some examples, the third blood pressure estimation may be an average of the first blood pressure estimation and the second blood pressure estimation. In some such examples, the third blood pressure estimation may be a weighted average of the first blood pressure estimation and the second blood pressure estimation. The average may, for example, be weighted according to the perceived reliability of the methods underlying the first blood pressure estimation and the second blood pressure estimation.

Figures 10A and 10B show examples of blocks of a data collection and heart rate waveform (HRW) determination process. At least some blocks of Figures 10A and 10B may, for example, be performed by the apparatus 200 of Figure 2 or by a similar apparatus. As with other methods disclosed herein, the method outlined in Figures 10A and 10B may include more or fewer blocks than indicated. Figures 10A and 10B are based on a prototype process that the present inventors implemented, but at least some blocks of Figures 10A and 10B may be used in a commercial implementation. Moreover, the blocks of methods disclosed herein are not necessarily performed in the order indicated. In some instances, one or more of the blocks shown in Figures 10A and 10B may be performed concurrently. In some examples, the "input hardware" that is referenced in block 1001 may include the version of apparatus 200 that is shown in Figures 5A and 5B.

In this implementation, block 1005 involves obtaining PAPG data from different elevations relative to a user's heart. According to this example, block 1005 involves obtaining a first set of PAPG data from a user's finger while the finger is at approximately the same elevation as a user's heart, obtaining a second set of PAPG data from the user's finger while the finger is at an elevation above the user's heart (e.g., with the user's arm extended above the user's head when the user is standing or seated in an upright position) and obtaining a third set of PAPG data from the user's finger while the finger is at an elevation below the user's heart (e.g., with the user's arm extended downward when the user is standing or seated in an upright position). The PAPG data from three different elevations relative to a user's heart correspond to the data structures labeled "Calibration Distance #1," "Calibration Distance #2" and "Calibration Distance #3" that are shown in block 1055.

In this example, a live or "real time" signal-to-noise ratio (SNR) and/or finger position checking process 1010 is performed concurrently with the data acquisition process 1005. According to this implementation, the output of a fast data formatting block 1011 is provided to the determination block 1013, in which SNR and/or a finger position may be evaluated. In some such examples, the determination block 1013 involves determining whether a HRW has been detected. In this example, the process continues to block 1015 if the determination block 1013 indicates a positive outcome, whereas a user prompt is provided in block if the process 1010 indicates a negative outcome.

The HRW generation block 1035 may involve one or more methods of HRW determination and generation. Block 1037 involves what is referred to herein as a "peak-to-peak" HRW generation process, which may proceed as described elsewhere herein. In this example, block 1039 involves HRW generation according to a Hilbert transform of detected acoustic waves corresponding to photoacoustic emissions from the blood and the blood vessels. The Hilbert transform returns a complex helical sequence, sometimes called the analytic signal, from a real data sequence. The signal contains a real part and imaginary part. The imaginary part is a version of the original real sequence with a 90° phase shift. Sines are therefore transformed to cosines, and conversely, cosines are transformed to sines. The Hilbert-transformed series has the same amplitude and frequency content as the original sequence. The transform includes phase information that depends on the phase of the original. The Hilbert transform is useful in calculating instantaneous attributes of a time series, especially the amplitude and the frequency. Block 1039 and other methods, which may include those of block 1041, involve an evaluation of the total energy returned in the signal corresponding to the detected acoustic waves. Some methods may involve an evaluation of the peak energy, whereas others may involve an integration or summation of the area under a curve represented by the detected acoustic waves. Some such methods may involve an absolute value trapezoidal detection technique , which is one method of approximating an integration or summation of the area under a curve. In some examples, the absolute value trapezoidal detection technique starts with a sinusoidal waveform with the y-axis centered around 0. An absolute value of the signal is determined, bringing any negative components/cycles positive or above 0. After the absolute value is determined, in some examples numerical integration is applied via the trapezoidal method. This method approximates the integration over an interval by breaking the area down into trapezoids with more easily computable areas. This absolute value trapezoidal detection technique is applied within the interval/window (corresponding to a depth range into the target, e.g., the finger) that is specified. In some implementations, instead of applying the absolute value trapezoidal detection technique, an absolute mean detection method may be applied. According to some such examples, the absolute mean detection method involves determining the mean of the absolute value of the signals in the interval/window/depth range of interest.

In the examples shown in Figures 10A and 10B, the HRW generation block 1035 involves the evaluation of, and the output of HRW data relating to, depth-discriminated data and depth-integrated data. The depth-discriminated data may be obtained by sampling the acoustic data via a plurality of RGDs in order to obtain data from a plurality of corresponding depths, e.g., as discussed elsewhere herein. The depth-integrated data may be obtained by receiving the acoustic data during a RGW that corresponds to a plurality of depths within the finger, producing an undifferentiated output that includes responses of multiple blood vessels, capillaries, etc. Accordingly, the depth-integrated data is similar to the data that would be obtained via a PPG process. In this implementation, HRWs are calculated based on both the depth-discriminated data and the depth-integrated data, and the HRWs are output from the HRW generation block 1035.

According to this example, the HRW generation block 1035 includes metadata with the outputted and saved HRW data. In this example, the metadata includes data corresponding to the person from whom the PAPG data has been acquired. Such metadata may include age data, weight data, height data, body mass index data, gender data, data regarding medication currently being taken and/or data regarding known health issues, particularly health issues that involve the heart and/or circulatory system, etc. The metadata may or may not be used for the purpose of blood pressure calculations, depending on the particular implementation.

In this example, the depth-discriminated HRW data is input to the automatic artery/vein HRW detection block 1045. In some examples, the automatic artery/vein HRW detection block 1045 may involve some or all of the processes that are described above with reference to Figures 7 and 8. In this implementation, the depth-discriminated HRW data is also input to the manual artery/vein HRW detection block 1043. In this example, the manual artery/vein HRW detection block 1043 involves a manual process of detecting artery and vein HRWs. The manual artery/vein HRW detection block 1043 has been used for determination of "ground truth" artery and vein HRWs during the developmental phases of the inventors' work. The manual artery/vein HRW detection block 1043 is not expected to be a necessary component of a commercial product.

According to this implementation, artery and vein diameter data are determined and output by the HRW generation block 1035. Parts of the artery or vein that do not change their optical absorption during a heart period will not exhibit a HRW, while those that do change their optical absorption during that time will exhibit a HRW. For example, the inner parts of an artery or vein may not change their optical absorption during the heart period. However, the outer parts, especially the regions that are just outside the blood vessel will suddenly encompass the outer part of the blood vessel as it distends during the HRW. This process changes the optical absorption. Some implementations have sufficient resolution and use sufficiently narrow time windows to distinguish blood vessels of various diameters. For example, an implementation having a receiver with a resolution of 0.25 mm and with a time window set to correspond with a tissue depth range of 0.25 mm can distinguish a 0.5mm diameter artery from a 1.0mm diameter artery. Some implementations leverage the same data to determine how much the blood vessels distend during a cardiac cycle. Accordingly, in this example artery and vein distention data are determined and output by the HRW generation block 1035.

After determining the artery and vein HRWs, in this example the artery-vein phase shift (AVPS) is calculated in block 1047. According to some implementations, a blood pressure estimation may subsequently be made that is based, at least in part, on AVPS data.

In block 1050, the AVPS data, metadata, depth-discriminated HRWs, depth-integrated HRWs, artery distention data, vein distention data, artery diameter data and vein diameter data are saved. Block 1055 represents a server location in which such data may be stored, as well as examples of data locations 1 and 2 in which the data may be stored.

Figure 11 shows examples of blocks of some disclosed methods. The blocks of Figure 11 (and those of other flow diagrams provided herein) may, for example, be performed by the apparatus 200 of Figure 2 or by a similar apparatus. As with other methods disclosed herein, the method outlined in Figure 11 may include more or fewer blocks than indicated. Moreover, the blocks of methods disclosed herein are not necessarily performed in the order indicated. In some instances, one or more of the blocks shown in Figure 11 may be performed concurrently.

In this example, block 1105 involves filtering input HRW data. In some examples, the input HRW data may be depth-integrated HRW data, whereas in other instances the input HRW data may be depth-discriminated HRW data. In this example, the original signal is noisy and includes respiration effects. According to this example, block 1105 involves applying a bandpass filter having a pass band of 0.1 Hz to 10 Hz to the input HRW data. Other examples may involve the application of bandpass filters having different pass bands. In this example, block 1105 involves applying a DC offset to at least some of the input HRW data, in order to remove the respiration effects.

In this implementation, filtered HRW data that is output from block 1105 is input to HRW averaging block 1110. HRW averaging can be beneficial due to the variability in HRWs from heartbeat to heartbeat, at least in part because averaging helps to remove random noise. According to some implementations, tens of seconds of filtered HRW data may be averaged in block 1105 (e.g., 10 seconds of filtered HRW data, 20 seconds of filtered HRW data, 30 seconds of filtered HRW data, 40 seconds of filtered HRW data, 50 seconds of filtered HRW data, 60 seconds of filtered HRW data, etc.).

According to this example, block 1115 involves HRW fiducial detection, including HRW peak and valley detection based on the averaged HRW data. In this example, block 1115 involves detecting systolic and diastolic valleys in the averaged HRW data. In this implementation, block 1120 involves HRW segmentation. According to this example, block 1120 involves segmentation of the averaged HRW data into individual HRWs, based at least in part on the output of the HRW fiducial detection of block 1115.

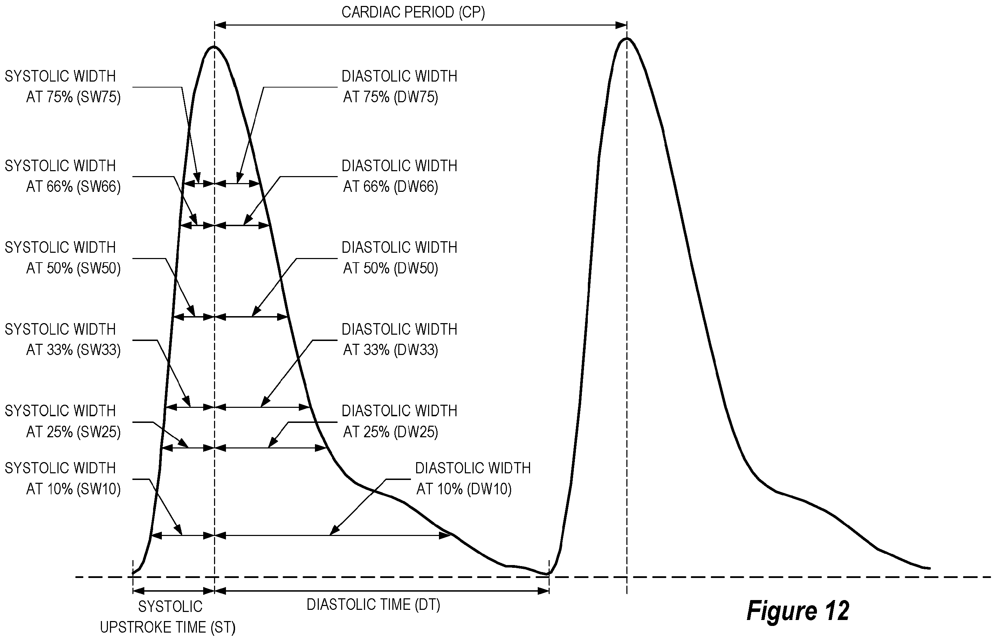

In this implementation, block 1125 involves extracting HRW features from the individual HRW segments output by block 1120. Examples of HRW features that may be extracted in block 1125 are illustrated in Figure 12 and are described below.

According to this example, block 1130 involves training a neural network to prepare a blood pressure estimate (illustrated as "BP Estimate A" in Figure 11), based on the extracted features output by block 1125. In some examples (but not all examples), metadata (e.g., metadata regarding the person from whom the original HRW signals were obtained) may also be input to the neural network. In some such examples, training the neural network may involve training the neural network to minimize a cost function based on a difference between (1) a "ground truth" blood pressure measurement for a person based on a technique known to be reliable (e.g., a catheter-based technique or a cuff-based technique, for persons who do not suffer from "white coat hypertension") and (2) a blood pressure estimate for the same person based on the extracted features output by block 1125. According to some examples, the neural network includes at least two hidden layers of neurons in addition to an input layer and an output layer. In some such examples, the number of neurons in the input layer corresponds to the number of input HRW features.

In some alternative implementations of the method 1100, block 1130 may involve the application of another type of artificial intelligence, such as a machine learning process, which may be a supervised learning process, an unsupervised learning process or a reinforcement learning process. In some such alternative implementations, block 1130 may involve the application of a Beyesian machine learning process, a linear regression process, a logistic regression process, etc.

In some alternative "run time" examples of the method 1100, a previously-trained neural network may provide a blood pressure estimate in block 1130 based, at least in part, on the extracted features output by block 1125.

Figure 12 shows examples of heart rate waveform (HRW) features that may be extracted according to some implementations of the method of Figure 11. The horizontal axis of Figure 12 represents time and the vertical axis represents signal amplitude. The cardiac period is indicated by the time between adjacent peaks of the HRW. The systolic and diastolic time intervals are indicated below the horizontal axis. During the systolic phase of the cardiac cycle, as a pulse propagates through a particular location along an artery, the arterial walls expand according to the pulse waveform and the elastic properties of the arterial walls. Along with the expansion is a corresponding increase in the volume of blood at the particular location or region, and with the increase in volume of blood an associated change in one or more characteristics in the region. Conversely, during the diastolic phase of the cardiac cycle, the blood pressure in the arteries decreases and the arterial walls contract. Along with the contraction is a corresponding decrease in the volume of blood at the particular location, and with the decrease in volume of blood an associated change in the one or more characteristics in the region.

The HRW features that are illustrated in Figure 12 pertain to the width of the systolic and/or diastolic portions of the HRW curve at various "heights," which are indicated by a percentage of the maximum amplitude. For example, the SW50 feature is the width of the systolic portion of the HRW curve at a "height" of 50% of the maximum amplitude. In some implementations, the HRW features used for blood pressure estimation may include some or all of the SW10, SW25, SW33, SW50, SW66, SW75, DW10, DW25, DW33, DW50, DW66 and DW75 HRW features. In other implementations, additional HRW features may be used for blood pressure estimation. Such additional HRW features may, in some instances, include the sum and ratio of the SW and DW at one or more "heights," e.g., (DW75 + SW75), DW75/SW75, (DW66 + SW66), DW66/SW66, (DW50 + SW50), DW50/SW50, (DW33 + SW33), DW33/SW33, (DW25 + SW25), DW25/SW25 and/or (DW10 + SW10), DW10/SW10. Other implementations may use yet other HRW features for blood pressure estimation. Such additional HRW features may, in some instances, include sums, differences, ratios and/or other operations based on more than one "height," such as (DW75 + SW75)/(DW50 + SW50), (DW50 + SW50/(DW10 + SW10), etc.

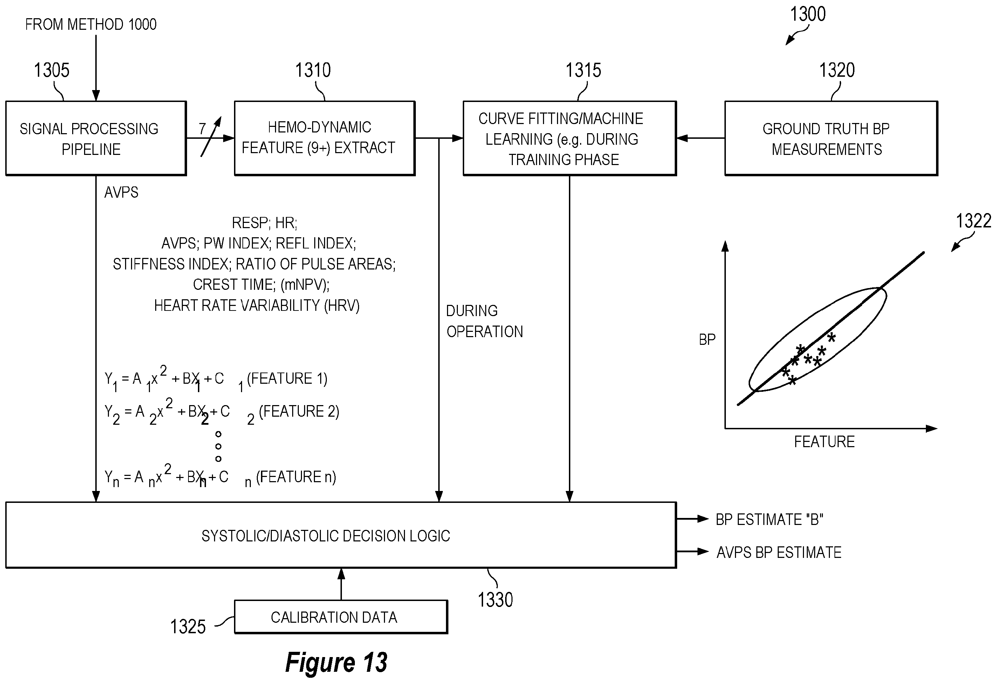

Figure 13 is a flow diagram that shows example blocks of making blood pressure estimates based on hemodynamic features. The blocks of Figure 13 (and those of other flow diagrams provided herein) may, for example, be performed by the apparatus 200 of Figure 2 or by a similar apparatus. As with other methods disclosed herein, the method outlined in Figure 13 may include more or fewer blocks than indicated. Moreover, the blocks of methods disclosed herein are not necessarily performed in the order indicated. In some instances, one or more of the blocks shown in Figure 13 may be performed concurrently.

According to this example, block 1305 involves obtaining various types of data that are described above with reference to Figures 10A and 10B. Such data may include artery HRW data, vein HRW data, superimposed HRW data (e.g., depth-integrated HRW data), artery diameter data, vein diameter data, artery depth data, vein depth data, AVPS data, etc.

The systolic/diastolic decision logic block 1330 may, in some examples, implement a machine learning process, which may be a supervised learning process, an unsupervised learning process or a reinforcement learning process. The systolic/diastolic decision logic block 1330 may, in some examples, implement a linear regression process. In other examples, the systolic/diastolic decision logic block 1330 may implement one or more other types of AI, such as such as a neural network.

During "run time" operation, in some examples the AVPS data may be provided directly to a trained systolic/diastolic decision logic block 1330. Other implementations may involve pre-processing of the AVPS data before it is provided to a trained systolic/diastolic decision logic block 1330. Such pre-processing may, for example, involve averaging, filtering, summing, determining minima and/or maxima, etc.

According to this example, the hemodynamic feature extraction block 1310 receives the data from block 1305 and determines a plurality of hemodynamic features. Such hemodynamic features may include Modified Normalized Pulse Volume (mNPV) data, respiration data, heart rate data, heart rate variability (HRV) data, blood vessel stiffness index data, ratio of pulse area data, crest time data, AVPS data, etc. In the foregoing, mNPV can be defined as the ratio of the peak to peak amplitude of the PAPG pulse to the DC component of the pulse, or as a function of said ratio; the blood vessel stiffness can be measured as a ratio of the person's height to a time delay between systolic and diastolic peak of the PAPG pulse; ratio of pulse area can be defined as the ratio of the area under PAPG pulse between the inflection point and the pulse end to the area under PAPG pulse between the pulse start and the inflection point; and the crest time can be measured as the time from the pulse start to the systolic peak. While these and other hemodynamic features do not measure the blood pressure directly, a relationship has been established between the hemodynamic features and systolic/diastolic blood pressure. A neural network can be trained to establish this relationship.

During "run time" operation, the n hemodynamic features extracted by the hemodynamic feature extraction block 1310 may be provided directly to a trained systolic/diastolic decision logic block 1330. In some examples, the objective of the training process is to find the relationship between the measured hemodynamic features and the blood pressure on a training dataset by minimizing the error between the predicted blood pressure and the ground truth blood pressure. There exist many methods for this. Some examples include linear or nonlinear regression, neural networks, Support Vector Machines (SVM), etc. During a training process that is represented by block 1315 in Figure 13, the *n* hemodynamic features are provided to block 1315, along with reliable "ground truth" blood pressure measurements from block 1320. As suggested by the graph 1322, in some examples training the systolic/diastolic decision logic block 1330 may involve separate linear regression analyses based on each of the n hemodynamic features, or, alternatively, on all hemodynamic features together. The polynomials Y₁ through Yₙ shown below the graph 1322 are merely presented by way of example. Some implementations may involve an analysis based one or more other types of functions, such as higher-order polynomial functions, Bessel functions, trigonometric functions, etc. During the training process that is represented by block 1315, a separate analysis (e.g., a linear regression analysis) may be performed that is based on the AVPS data and the "ground truth" blood pressure measurements from block 1320.

In the example shown in Figure 13, a trained systolic/diastolic decision logic block 1330 is configured to provide one blood pressure estimate based on the *n* hemodynamic features extracted by the hemodynamic feature extraction block 1310 and another blood pressure estimate based on the AVPS data. In this example, the blood pressure estimates are also based on calibration data 1325. In some examples, the calibration data 1325 may include blood pressure measurements from a cuff-type blood pressure measurement device or a catheter-type blood pressure measurement device. Alternatively, or additionally, in some implementations, the calibration data 1325 may include blood pressure estimates based on two or more elevations of a body part from which ultrasonic data are being obtained, e.g., two or more elevations of an arm when ultrasonic data are being obtained from a corresponding digit or wrist. Some such implementations may also involve obtaining data from a sensor that can measure, or estimate, the height of the apparatus by which ultrasonic data are being obtained (e.g., an implementation of the apparatus 200 disclosed herein) relative to a subject's heart. In some examples, such a height-estimating device may be based, at least in part, on input from an optical sensor/camera. Some such examples may incorporate a trained neural network, or another implementation of artificial intelligence, configured for estimating the height of the apparatus by which ultrasonic data are being obtained relative to the subject's heart. In some examples, a height-estimating device may be based, at least in part, on input from a manometer or an atmospheric pressure gauge. Alternatively, or additionally, a height-estimating device may be based, at least in part, on input from one or more accelerometers. In some such implementations, an accelerometer may be attached to a subject's upper arm, with the accelerometer's axis aligned along the long axis of the humerus. Alternatively, or additionally, some such implementations may include an accelerometer attached to the subject's wrist or finger. In some alternative implementations, a subject may be instructed to hold the subject's arm in two or more specific positions, e.g., one position in which the subject's arm is relaxing along the side of the subject's body and another position in which the subject's arm is extended at shoulder height.

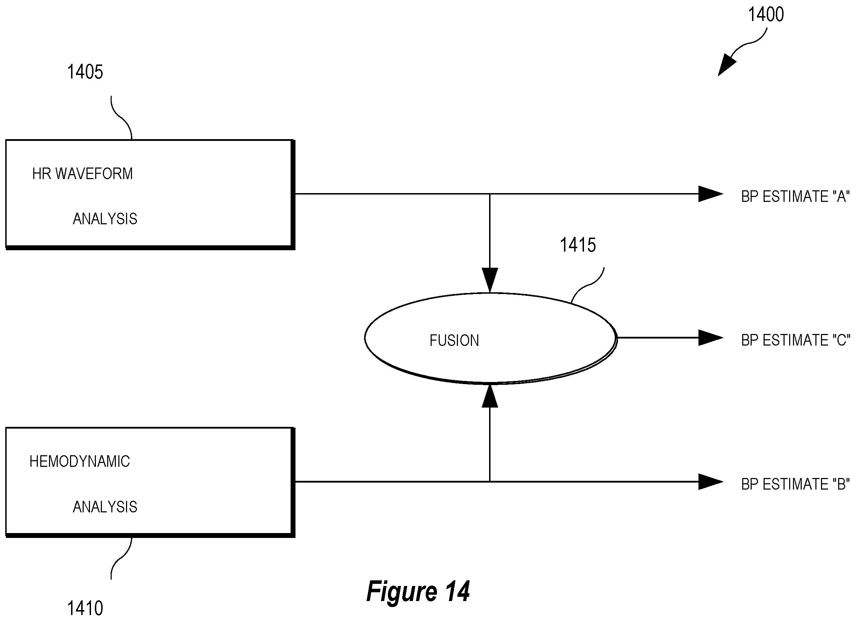

Figure 14 is a flow diagram that provides an example of making a blood pressure estimation that is based on both an HRW analysis and a hemodynamic analysis. The blocks of Figure 14 (and those of other flow diagrams provided herein) may, for example, be performed by the apparatus 200 of Figure 2 or by a similar apparatus. As with other methods disclosed herein, the method outlined in Figure 14 may include more or fewer blocks than indicated. Moreover, the blocks of methods disclosed herein are not necessarily performed in the order indicated. In some instances, one or more of the blocks shown in Figure 14 may be performed concurrently.

In this example, block 1405 involves making a blood pressure estimate (BP Estimate "A") based on HRW analysis. Block 1405 may, for example, involve at least some of the operations that are described above with reference to Figure 11, or similar processes.

In this example, block 1410 involves making another blood pressure estimate (BP Estimate "B") based on a hemodynamic analysis. Block 1410 may, for example, involve at least some of the operations that are described above with reference to Figure 13, or similar processes. In some examples, BP Estimate B may be based on multiple hemodynamic features, whereas in other examples BP Estimate B may be based only on AVPS data and calibration data.

According to this example, block 1415 involves making a third blood pressure estimate (BP Estimate "C") based on BP Estimate A and BP Estimate B. In some such examples, BP Estimate C may be an average (e.g., a weighted average) of BP Estimate A and BP Estimate B. The weighting of a weighted average may be based on the relative accuracy of the blood pressure estimate based on the HRW analysis, as compared to the accuracy of the blood pressure estimate based on the hemodynamic analysis. For example, if BP Estimate A is believed to be twice as accurate as BP Estimate B, the weighting of BP Estimate A may be 2X that of BP Estimate B. In one such example, if BP Estimate A were 120/80 and BP Estimate B were 126/80, BP Estimate C may be ((120 + 120 + 126)/3 = 122)/80.

In other examples, block 1415 may involve making BP Estimate C based on a combination or fusion of the methods that were used to produce BP Estimate A and BP Estimate B. There are many possible approaches for the fusion of different methods. In some examples, the BP Estimate C may be based on a neural network trained to make the estimate based on both HRW features and hemodynamic features. This type of fusion may be referred to as fusion on a features level. In some alternative examples, the fusion may be done on the results level. In this case, the predicted systolic and diastolic blood pressure from different methods may be combined to output the resulting blood pressure, e.g., as outlined in the simple example above.

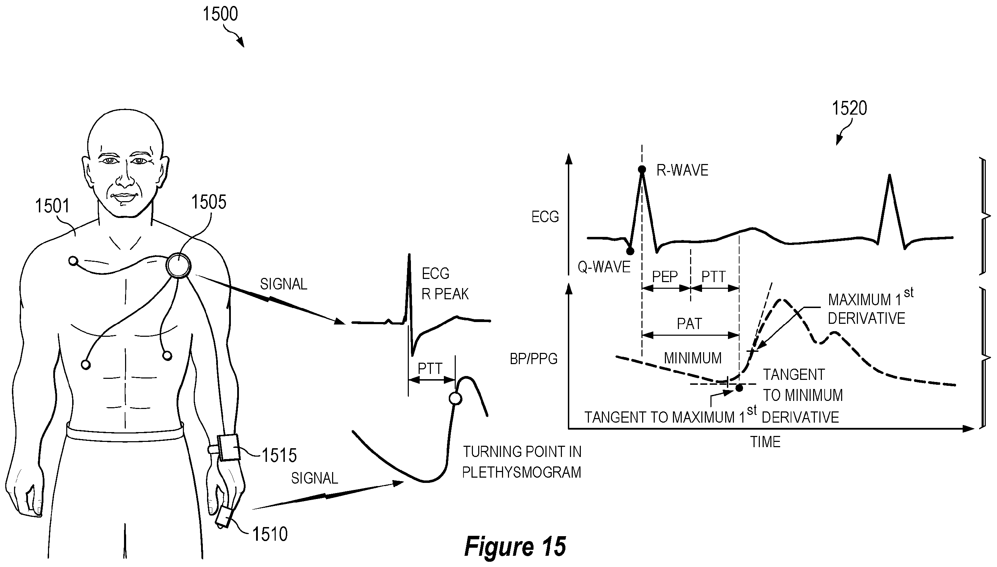

Figure 15 shows examples of devices that may be used in a system for estimating blood pressure based, at least in part, on pulse transit time (PTT). As with other figures provided herein, the numbers, types and arrangements of elements are merely presented by way of example. According to this example, the system 1500 includes at least two sensors. In this example, the system 1500 includes at least an electrocardiogram sensor 1505 and a device 1510 that is configured to be mounted on a finger of the person 1501. In this example, the device 1510 is, or includes, an apparatus configured to perform at least some PAPG methods disclosed herein. For example, the device 1510 may be, or may include, the apparatus 200 of Figure 2 or a similar apparatus.

As noted in the graph 1520, the PAT includes two components, the pre-ejection period (PEP, the time needed to convert the electrical signal into a mechanical pumping force and isovolumetric contraction to open the aortic valves) and the PTT. The starting time for the PAT can be estimated based on the QRS complex-an electrical signal characteristic of the electrical stimulation of the heart ventricles. As shown by the graph 1520, in this example the beginning of a pulse arrival time (PAT) may be calculated according to an R-Wave peak measured by the electrocardiogram sensor 1505 and the end of the PAT may be detected via analysis of signals provided by the device 1510. In this example, the end of the PAT is assumed to correspond with an intersection between a tangent to a local minimum value detected by the device 1510 and a tangent to a maximum slope/first derivative of the sensor signals after the time of the minimum value.

There are many known algorithms for blood pressure estimation based on the PTT and/or the PAT, some of which are summarized in Table 1 and described in the corresponding text on pages 5-10 of Sharma, M., et al., Cuff-Less and Continuous Blood Pressure Monitoring: a Methodological Review ("Sharma"), in Multidisciplinary Digital Publishing Institute (MDPI) Technologies 2017, 5, 21.

Some previously-disclosed methods have involved calculating blood pressure according to one or more of the equations shown in Table 1 of Sharma, or other known equations, based on a PTT and/or PAT measured by a sensor system that includes a PPG sensor. As noted above, some disclosed PAPG-based implementations are configured to distinguish artery HRWs from other HRWs. Such implementations may provide more accurate measurements of the PTT and/or PAT, relative to those measured by a PPG sensor. Therefore, disclosed PAPG-based implementations may provide more accurate blood pressure estimations, even when the blood pressure estimations are based on previously-known formulae.

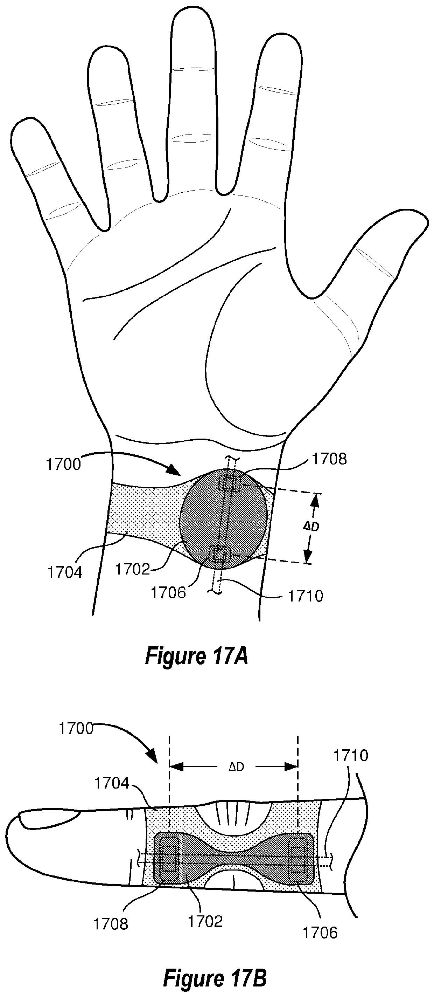

Other implementations of the system 1500 may not include the electrocardiogram sensor 1505. In some such implementations, the device 1515, which is configured to be mounted on a wrist of the person 1501, may be, or may include, an apparatus configured to perform at least some PAPG methods disclosed herein. For example, the device 1515 may be, or may include, the apparatus 200 of Figure 2 or a similar apparatus. According to some such examples, the device 1515 may include a light source system and two or more ultrasonic receivers. One example is described below with reference to Figure 17A. In some examples, the device 1515 may include an array of ultrasonic receivers.

In some implementations of the system 1500 that do not include the electrocardiogram sensor 1505, the device 1510 may include a light source system and two or more ultrasonic receivers. One example is described below with reference to Figure 17B.

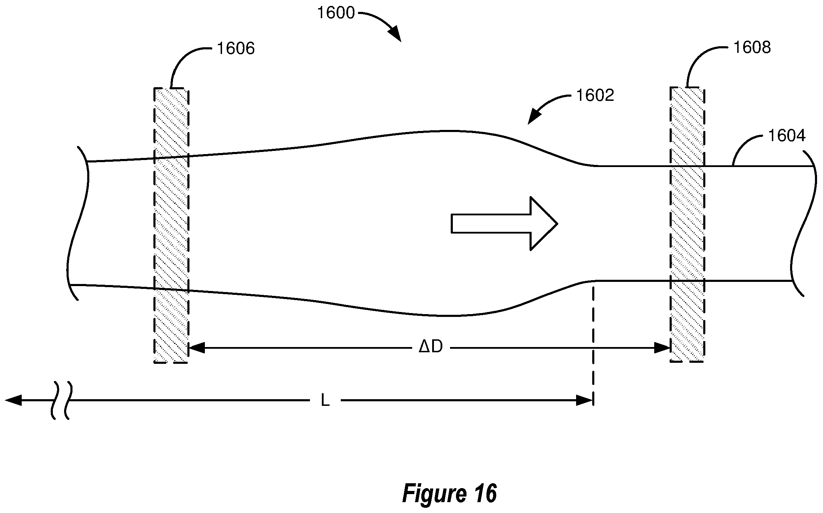

Figure 16 shows a cross-sectional side view of a diagrammatic representation of a portion of an artery 1600 through which a pulse 1602 is propagating. The block arrow in Figure 16 shows the direction of blood flow and pulse propagation. As diagrammatically shown, the propagating pulse 1602 causes strain in the arterial walls 1604, which is manifested in the form of an enlargement in the diameter (and consequently the cross-sectional area) of the arterial walls-referred to as "distension." The spatial length L of an actual propagating pulse along an artery (along the direction of blood flow) is typically comparable to the length of a limb, such as the distance from a subject's shoulder to the subject's wrist or finger, and is generally less than one meter (m). However, the length L of a propagating pulse can vary considerably from subject to subject, and for a given subject, can vary significantly over durations of time depending on various factors. The spatial length L of a pulse will generally decrease with increasing distance from the heart until the pulse reaches capillaries.

As described above, some particular implementations relate to devices, systems and methods for estimating blood pressure or other cardiovascular characteristics based on estimates of an arterial distension waveform. The terms "estimating," "measuring," "calculating," "inferring," "deducing," "evaluating," "determining" and "monitoring" may be used interchangeably herein where appropriate unless otherwise indicated. Similarly, derivations from the roots of these terms also are used interchangeably where appropriate; for example, the terms "estimate," "measurement," "calculation," "inference" and "determination" also are used interchangeably herein. In some implementations, the pulse wave velocity (PWV) of a propagating pulse may be estimated by measuring the pulse transit time (PTT) of the pulse as it propagates from a first physical location along an artery to another more distal second physical location along the artery. It will be appreciated that this PTT is different from the PTT that is described above with reference to Figure 15. However, either version of the PTT may be used for the purpose of blood pressure estimation. Assuming that the physical distance *ΔD* between the first and the second physical locations is ascertainable, the PWV can be estimated as the quotient of the physical spatial distance *ΔD* traveled by the pulse divided by the time (PTT) the pulse takes in traversing the physical spatial distance *ΔD.* Generally, a first sensor positioned at the first physical location is used to determine a starting time (also referred to herein as a "first temporal location") at which point the pulse arrives at or propagates through the first physical location. A second sensor at the second physical location is used to determine an ending time (also referred to herein as a "second temporal location") at which point the pulse arrives at or propagates through the second physical location and continues through the remainder of the arterial branch. In such examples, the PTT represents the temporal distance (or time difference) between the first and the second temporal locations (the starting and the ending times).

The fact that measurements of the arterial distension waveform are performed at two different physical locations implies that the estimated PWV inevitably represents an average over the entire path distance *ΔD* through which the pulse propagates between the first physical location and the second physical location. More specifically, the PWV generally depends on a number of factors including the density of the blood *ρ*, the stiffness *E* of the arterial wall (or inversely the elasticity), the arterial diameter, the thickness of the arterial wall, and the blood pressure. Because both the arterial wall elasticity and baseline resting diameter (for example, the diameter at the end of the ventricular diastole period) vary significantly throughout the arterial system, PWV estimates obtained from PTT measurements are inherently average values (averaged over the entire path length *ΔD* between the two locations where the measurements are performed).

In traditional methods for obtaining PWV, the starting time of the pulse has been obtained at the heart using an electrocardiogram (ECG) sensor, which detects electrical signals from the heart. For example, the starting time can be estimated based on the QRS complex-an electrical signal characteristic of the electrical stimulation of the heart ventricles. In such approaches, the ending time of the pulse is typically obtained using a different sensor positioned at a second location (for example, a finger). As a person having ordinary skill in the art will appreciate, there are numerous arterial discontinuities, branches, and variations along the entire path length from the heart to the finger. The PWV can change by as much as or more than an order of magnitude along various stretches of the entire path length from the heart to the finger. As such, PWV estimates based on such long path lengths are unreliable.

In various implementations described herein, PTT estimates are obtained based on measurements (also referred to as "arterial distension data" or more generally as "sensor data") associated with an arterial distension signal obtained by each of a first arterial distension sensor 1606 and a second arterial distension sensor 1608 proximate first and second physical locations, respectively, along an artery of interest. In some particular implementations, the first arterial distension sensor 1606 and the second arterial distension sensor 1608 are advantageously positioned proximate first and second physical locations between which arterial properties of the artery of interest, such as wall elasticity and diameter, can be considered or assumed to be relatively constant. In this way, the PWV calculated based on the PTT estimate is more representative of the actual PWV along the particular segment of the artery. In turn, the blood pressure P estimated based on the PWV is more representative of the true blood pressure. In some implementations, the magnitude of the distance *ΔD* of separation between the first arterial distension sensor 1606 and the second arterial distension sensor 1608 (and consequently the distance between the first and the second locations along the artery) can be in the range of about 1 centimeter (cm) to tens of centimeters-long enough to distinguish the arrival of the pulse at the first physical location from the arrival of the pulse at the second physical location, but close enough to provide sufficient assurance of arterial consistency. In some specific implementations, the distance *ΔD* between the first and the second arterial distension sensors 1606 and 1608 can be in the range of about 1 cm to about 30 cm, and in some implementations, less than or equal to about 20 cm, and in some implementations, less than or equal to about 10 cm, and in some specific implementations less than or equal to about 5 cm. In some other implementations, the distance *ΔD* between the first and the second arterial distension sensors 1606 and 1608 can be less than or equal to 1 cm, for example, about 0.1 cm, about 0.25 cm, about 0.5 cm or about 0.75 cm. By way of reference, a typical PWV can be about 15 meters per second (m/s). Using an ambulatory monitoring device in which the first and the second arterial distension sensors 1606 and 1608 are separated by a distance of about 5 cm, and assuming a PWV of about 15 m/s implies a PTT of approximately 3.3 milliseconds (ms).

The value of the magnitude of the distance *ΔD* between the first and the second arterial distension sensors 1606 and 1608, respectively, can be preprogrammed into a memory within a monitoring device that incorporates the sensors (for example, such as a memory of, or a memory configured for communication with, the control system 206 that is described above with reference to Figure 2). As will be appreciated by a person of ordinary skill in the art, the spatial length *L* of a pulse can be greater than the distance *ΔD* from the first arterial distension sensor 1606 to the second arterial distension sensor 1608 in such implementations. As such, although the diagrammatic pulse 1602 shown in Figure 16 is shown as having a spatial length *L* comparable to the distance between the first arterial distension sensor 1606 and the second arterial distension sensor 1608, in actuality each pulse can typically have a spatial length *L* that is greater and even much greater than (for example, about an order of magnitude or more than) the distance *ΔD* between the first and the second arterial distension sensors 1606 and 1608.

### Sensing Architecture and Topology

In some implementations of the ambulatory monitoring devices disclosed herein, both the first arterial distension sensor 1606 and the second arterial distension sensor 1608 are sensors of the same sensor type. In some such implementations, the first arterial distension sensor 1606 and the second arterial distension sensor 1608 are identical sensors. In such implementations, each of the first arterial distension sensor 1606 and the second arterial distension sensor 1608 utilizes the same sensor technology with the same sensitivity to the arterial distension signal caused by the propagating pulses, and has the same time delays and sampling characteristics. In some implementations, each of the first arterial distension sensor 1606 and the second arterial distension sensor 1608 is configured for photoacoustic plethysmography (PAPG) sensing, e.g., as disclosed elsewhere herein. Some such implementations include a light source system and two or more ultrasonic receivers, which may be instances of the light source system 204 and the ultrasonic receiver 202 of Figure 2. In some implementations, each of the first arterial distension sensor 1606 and the second arterial distension sensor 1608 is configured for ultrasound sensing via the transmission of ultrasonic signals and the receipt of corresponding reflections. In some alternative implementations, each of the first arterial distension sensor 1606 and the second arterial distension sensor 1608 may be configured for impedance plethysmography (IPG) sensing, also referred to in biomedical contexts as bioimpedance sensing. In various implementations, whatever types of sensors are utilized, each of the first and the second arterial distension sensors 1606 and 1608 broadly functions to capture and provide arterial distension data indicative of an arterial distension signal resulting from the propagation of pulses through a portion of the artery proximate to which the respective sensor is positioned. For example, the arterial distension data can be provided from the sensor to a processor in the form of voltage signal generated or received by the sensor based on an ultrasonic signal or an impedance signal sensed by the respective sensor.

As described above, during the systolic phase of the cardiac cycle, as a pulse propagates through a particular location along an artery, the arterial walls expand according to the pulse waveform and the elastic properties of the arterial walls. Along with the expansion is a corresponding increase in the volume of blood at the particular location or region, and with the increase in volume of blood an associated change in one or more characteristics in the region. Conversely, during the diastolic phase of the cardiac cycle, the blood pressure in the arteries decreases and the arterial walls contract. Along with the contraction is a corresponding decrease in the volume of blood at the particular location, and with the decrease in volume of blood an associated change in the one or more characteristics in the region.

In the context of bioimpedance sensing (or impedance plethysmography), the blood in the arteries has a greater electrical conductivity than that of the surrounding or adjacent skin, muscle, fat, tendons, ligaments, bone, lymph or other tissues. The susceptance (and thus the permittivity) of blood also is different from the susceptances (and permittivities) of the other types of surrounding or nearby tissues. As a pulse propagates through a particular location, the corresponding increase in the volume of blood results in an increase in the electrical conductivity at the particular location (and more generally an increase in the admittance, or equivalently a decrease in the impedance). Conversely, during the diastolic phase of the cardiac cycle, the corresponding decrease in the volume of blood results in an increase in the electrical resistivity at the particular location (and more generally an increase in the impedance, or equivalently a decrease in the admittance).

A bioimpedance sensor generally functions by applying an electrical excitation signal at an excitation carrier frequency to a region of interest via two or more input electrodes, and detecting an output signal (or output signals) via two or more output electrodes. In some more specific implementations, the electrical excitation signal is an electrical current signal injected into the region of interest via the input electrodes. In some such implementations, the output signal is a voltage signal representative of an electrical voltage response of the tissues in the region of interest to the applied excitation signal. The detected voltage response signal is influenced by the different, and in some instances time-varying, electrical properties of the various tissues through which the injected excitation current signal is passed. In some implementations in which the bioimpedance sensor is operable to monitor blood pressure, heartrate or other cardiovascular characteristics, the detected voltage response signal is amplitude- and phase-modulated by the time-varying impedance (or inversely the admittance) of the underlying arteries, which fluctuates synchronously with the user's heartbeat as described above. To determine various biological characteristics, information in the detected voltage response signal is generally demodulated from the excitation carrier frequency component using various analog or digital signal processing circuits, which can include both passive and active components.

In some examples incorporating ultrasound sensors, measurements of arterial distension may involve directing ultrasonic waves into a limb towards an artery, for example, via one or more ultrasound transducers. Such ultrasound sensors also are configured to receive reflected waves that are based, at least in part, on the directed waves. The reflected waves may include scattered waves, specularly reflected waves, or both scattered waves and specularly reflected waves. The reflected waves provide information about the arterial walls, and thus the arterial distension.

In some implementations, regardless of the type of sensors utilized for the first arterial distension sensor 1606 and the second arterial distension sensor 1608, both the first arterial distension sensor 1606 and the second arterial distension sensor 1608 can be arranged, assembled or otherwise included within a single housing of a single ambulatory monitoring device. As described above, the housing and other components of the monitoring device can be configured such that when the monitoring device is affixed or otherwise physically coupled to a subject, both the first arterial distension sensor 1606 and the second arterial distension sensor 1608 are in contact with or in close proximity to the skin of the user at first and second locations, respectively, separated by a distance *ΔD,* and in some implementations, along a stretch of the artery between which various arterial properties can be assumed to be relatively constant. In various implementations, the housing of the ambulatory monitoring device is a wearable housing or is incorporated into or integrated with a wearable housing. In some specific implementations, the wearable housing includes (or is connected with) a physical coupling mechanism for removable non-invasive attachment to the user. The housing can be formed using any of a variety of suitable manufacturing processes, including injection molding and vacuum forming, among others. In addition, the housing can be made from any of a variety of suitable materials, including, but not limited to, plastic, metal, glass, rubber and ceramic, or combinations of these or other materials. In particular implementations, the housing and coupling mechanism enable full ambulatory use. In other words, some implementations of the wearable monitoring devices described herein are noninvasive, not physically-inhibiting and generally do not restrict the free uninhibited motion of a subject's arms or legs, enabling continuous or periodic monitoring of cardiovascular characteristics such as blood pressure even as the subject is mobile or otherwise engaged in a physical activity. As such, the ambulatory monitoring device facilitates and enables long-term wearing and monitoring (for example, over days, weeks or a month or more without interruption) of one or more biological characteristics of interest to obtain a better picture of such characteristics over extended durations of time, and generally, a better picture of the user's health.

In some implementations, the ambulatory monitoring device can be positioned around a wrist of a user with a strap or band, similar to a watch or fitness/activity tracker. Figure 17A shows an example ambulatory monitoring device 1700 designed to be worn around a wrist according to some implementations. In the illustrated example, the monitoring device 1700 includes a housing 1702 integrally formed with, coupled with or otherwise integrated with a wristband 1704. The first and the second arterial distension sensors 1706 and 1708 may, in some instances, each include an instance of the ultrasonic receiver 202 and a portion of the light source system 204 that are described above with reference to Figure 2. In this example, the ambulatory monitoring device 1700 is coupled around the wrist such that the first and the second arterial distension sensors 1706 and 1708 within the housing 1702 are each positioned along a segment of the radial artery 1710 (note that the sensors are generally hidden from view from the external or outer surface of the housing facing the subject while the monitoring device is coupled with the subject, but exposed on an inner surface of the housing to enable the sensors to obtain measurements through the subject's skin from the underlying artery). Also as shown, the first and the second arterial distension sensors 1706 and 1708 are separated by a fixed distance *ΔD.* In some other implementations, the ambulatory monitoring device 1700 can similarly be designed or adapted for positioning around a forearm, an upper arm, an ankle, a lower leg, an upper leg, or a finger (all of which are hereinafter referred to as "limbs") using a strap or band.

Figure 17B shows an example ambulatory monitoring device 1700 designed to be worn around a finger according to some implementations. The first and the second arterial distension sensors 1706 and 1708 may, in some instances, each include an instance of the ultrasonic receiver 202 and a portion of the light source system 204 that are described above with reference to Figure 2.

In some other implementations, the ambulatory monitoring devices disclosed herein can be positioned on a region of interest of the user without the use of a strap or band. For example, the first and the second arterial distension sensors 1706 and 1708 and other components of the monitoring device can be enclosed in a housing that is secured to the skin of a region of interest of the user using an adhesive or other suitable attachment mechanism (an example of a "patch" monitoring device).

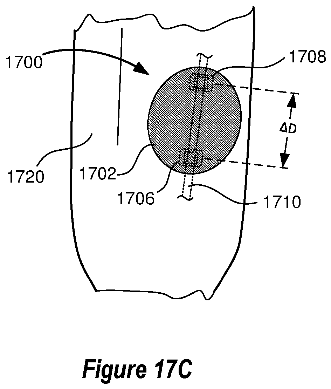

Figure 17C shows an example ambulatory monitoring device 1700 designed to reside on an earbud according to some implementations. According to this example, the ambulatory monitoring device 1700 is coupled to the housing of an earbud 1720. The first and second arterial distension sensors 1706 and 1708 may, in some instances, each include an instance of the ultrasonic receiver 202 and a portion of the light source system 204 that are described above with reference to Figure 2.

The various illustrative logics, logical blocks, modules, circuits and algorithm processes described in connection with the implementations disclosed herein may be implemented as electronic hardware, computer software, or combinations of both. The interchangeability of hardware and software has been described generally, in terms of functionality, and illustrated in the various illustrative components, blocks, modules, circuits and processes described above. Whether such functionality is implemented in hardware or software depends upon the particular application and design constraints imposed on the overall system.

The hardware and data processing apparatus used to implement the various illustrative logics, logical blocks, modules and circuits described in connection with the aspects disclosed herein may be implemented or performed with a general purpose single- or multi-chip processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general purpose processor may be a microprocessor, or, any conventional processor, controller, microcontroller, or state machine. A processor also may be implemented as a combination of computing devices, e.g., a combination of a DSP and a microprocessor, a plurality of microprocessors, one or more microprocessors in conjunction with a DSP core, or any other such configuration. In some implementations, particular processes and methods may be performed by circuitry that is specific to a given function.

In one or more aspects, the functions described may be implemented in hardware, digital electronic circuitry, computer software, firmware, including the structures disclosed in this specification and their structural equivalents thereof, or in any combination thereof. Implementations of the subject matter described in this specification also may be implemented as one or more computer programs, i.e., one or more modules of computer program instructions, encoded on a computer storage media for execution by, or to control the operation of, data processing apparatus.

If implemented in software, the functions may be stored on or transmitted over as one or more instructions or code on a computer-readable medium, such as a non-transitory medium. The processes of a method or algorithm disclosed herein may be implemented in a processor-executable software module which may reside on a computer-readable medium. Computer-readable media include both computer storage media and communication media including any medium that may be enabled to transfer a computer program from one place to another. Storage media may be any available media that may be accessed by a computer. By way of example, and not limitation, non-transitory media may include RAM, ROM, EEPROM, CD-ROM or other optical disk storage, magnetic disk storage or other magnetic storage devices, or any other medium that may be used to store desired program code in the form of instructions or data structures and that may be accessed by a computer. Also, any connection may be properly termed a computer-readable medium. Disk and disc, as used herein, includes compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and instructions on a machine readable medium and computer-readable medium, which may be incorporated into a computer program product.

Various modifications to the implementations described in this disclosure may be readily apparent to those having ordinary skill in the art, and the generic principles defined herein may be applied to other implementations without departing from the scope of this disclosure. Thus, the disclosure is not intended to be limited to the implementations shown herein, but is to be accorded the widest scope consistent with the claims, the principles and the novel features disclosed herein. The word "exemplary" is used exclusively herein, if at all, to mean "serving as an example, instance, or illustration." Any implementation described herein as "exemplary" is not necessarily to be construed as preferred or advantageous over other implementations.

Certain features that are described in this specification in the context of separate implementations also may be implemented in combination in a single implementation. Conversely, various features that are described in the context of a single implementation also may be implemented in multiple implementations separately or in any suitable subcombination. Moreover, although features may be described above as acting in certain combinations and even initially claimed as such, one or more features from a claimed combination may in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the implementations described above should not be understood as requiring such separation in all implementations, and it should be understood that the described program components and systems may generally be integrated together in a single software product or packaged into multiple software products. Additionally, other implementations are within the scope of the following claims. In some cases, the actions recited in the claims may be performed in a different order and still achieve desirable results.

It will be understood that unless features in any of the particular described implementations are expressly identified as incompatible with one another or the surrounding context implies that they are mutually exclusive and not readily combinable in a complementary and/or supportive sense, the totality of this disclosure contemplates and envisions that specific features of those complementary implementations may be selectively combined to provide one or more comprehensive, but slightly different, technical solutions. It will therefore be further appreciated that the above description has been given by way of example only and that modifications in detail may be made within the scope of this disclosure.

Various modifications to the implementations described in this disclosure may be readily apparent to those skilled in the art, and the generic principles defined herein may be applied to other implementations without departing from the scope of this disclosure. Thus, the following claims are not intended to be limited to the implementations shown herein, but are to be accorded the widest scope consistent with this disclosure, the principles and the novel features disclosed herein.

Additionally, certain features that are described in this specification in the context of separate implementations also can be implemented in combination in a single implementation. Conversely, various features that are described in the context of a single implementation also can be implemented in multiple implementations separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. Further, the drawings may schematically depict one more example processes in the form of a flow diagram. However, other operations that are not depicted can be incorporated in the example processes that are schematically illustrated. For example, one or more additional operations can be performed before, after, simultaneously, or between any of the illustrated operations. Moreover, various ones of the described and illustrated operations can itself include and collectively refer to a number of sub-operations. For example, each of the operations described above can itself involve the execution of a process or algorithm. Furthermore, various ones of the described and illustrated operations can be combined or performed in parallel in some implementations. Similarly, the separation of various system components in the implementations described above should not be understood as requiring such separation in all implementations. As such, other implementations are within the scope of the following claims. In some cases, the actions recited in the claims can be performed in a different order and still achieve desirable results.

## Claims

1. A biometric system, comprising:
a piezoelectric receiver;
a light source system configured for emitting a plurality of light pulses at a pulse repetition frequency between 10Hz and 1 MHz; and
a control system configured for:
controlling (305) the light source system to emit a plurality of light pulses into biological tissue at the pulse repetition frequency, the biological tissue including blood and blood vessels at depths within the biological tissue;
receiving (310) signals from the piezoelectric receiver corresponding to acoustic waves emitted from portions of the biological tissue, the acoustic waves corresponding to photoacoustic emissions from the blood and the blood vessels caused by the plurality of light pulses;
detecting (315) heart rate waveforms in the signals;
determining (320) a first subset of detected heart rate waveforms corresponding to vein heart rate waveforms; and
determining (325) a second subset of detected heart rate waveforms corresponding to artery heart rate waveforms,
wherein determining the first and second subsets of detected heart rate waveforms as vein heart rate waveforms and artery heart rate waveforms, respectively, involves determining, after a time of each light pulse of the plurality of light pulses, peak-to-peak values of the signals during range gate windows (RGW) of the same time duration but after range gate durations (RGD) of different time durations.

2. The biometric system of claim 1, wherein the control system is further configured for:
extracting heart rate waveform features from the heart rate waveforms; and
making a blood pressure estimation based, at least in part, on extracted heart rate waveform features.

3. The biometric system of claim 1, wherein receiving the signals from the piezoelectric receiver involves obtaining depth-discriminated signals by applying first through *N^{th}* acquisition time delays and receiving first through *N^{th}* signals during first through *N^{th}* acquisition time windows, each of the first through *N^{th}* acquisition time windows occurring after a corresponding one of the first through *N^{th}* acquisition time delays, wherein *N* is an integer greater than one, wherein the control system is configured for determining the first subset of detected heart rate waveforms and the second subset of detected heart rate waveforms based, at least in part, on the depth-discriminated signals.

4. The biometric system of claim 1, wherein the control system is further configured for:
extracting a set of hemodynamic features from the second subset of detected heart rate waveforms; and
making a first blood pressure estimation based, at least in part, on the set of hemodynamic features.

5. The biometric system of claim 4, wherein the control system is further configured for:
determining artery-vein phase shift, AVPS, data from the first subset of detected heart rate waveforms and the second subset of detected heart rate waveforms; and
making the first blood pressure estimation based, at least in part, on the AVPS data.

6. The biometric system of claim 5, wherein the control system is further configured for:
extracting heart rate waveform features from the heart rate waveforms;
making a second blood pressure estimation based, at least in part, on extracted heart rate waveform features; and
making a third blood pressure estimation based, at least in part, on the first blood pressure estimation and the second blood pressure estimation.

7. The biometric system of claim 1, wherein the control system is further configured for:
determining artery-vein phase shift, AVPS, data from the heart rate waveforms; and
making a first blood pressure estimation based, at least in part, on the AVPS data.

8. The biometric system of claim 7, wherein the control system is further configured for:
extracting heart rate waveform features from the heart rate waveforms;
making a second blood pressure estimation based, at least in part, on extracted heart rate waveform features; and
making a third blood pressure estimation based, at least in part, on the first blood pressure estimation and the second blood pressure estimation.

9. A biometric method, comprising:
controlling, via a control system, a light source system to emit a plurality of light pulses into biological tissue at a pulse repetition frequency, the biological tissue including blood and blood vessels at depths within the biological tissue;
receiving, by the control system, signals from a piezoelectric receiver corresponding to acoustic waves emitted from portions of the biological tissue, the acoustic waves corresponding to photoacoustic emissions from the blood and the blood vessels caused by the plurality of light pulses;
detecting, by the control system, heart rate waveforms in the signals;
determining, by the control system, a first subset of detected heart rate waveforms corresponding to vein heart rate waveforms; and
determining, by the control system, a second subset of detected heart rate waveforms corresponding to artery heart rate waveforms,
wherein determining the first and second subsets of detected heart rate waveforms as vein heart rate waveforms and artery heart rate waveforms, respectively, involves determining, after a time of each light pulse of the plurality of
light pulses, peak-to-peak values of the signals during range gate windows (RGW) of the same time duration but after range gate durations (RGD) of different time durations.

10. The biometric method of claim 9, further comprising:
extracting, by the control system, heart rate waveform features from the heart rate waveforms; and
making, by the control system, a blood pressure estimation based, at least in part, on extracted heart rate waveform features.

11. The biometric method of claim 9, wherein receiving the signals from the piezoelectric receiver involves obtaining depth-discriminated signals by applying first through *N^{th}* acquisition time delays and receiving first through *N^{th}* signals during first through *N^{th}* acquisition time windows, each of the first through *N^{th}* acquisition time windows occurring after a corresponding one of the first through *N^{th}* acquisition time delays, wherein *N* is an integer greater than one, the method optionally further comprising determining, by the control system, the first subset of detected heart rate waveforms and the second subset of detected heart rate waveforms based, at least in part, on the depth-discriminated signals.

12. The biometric method of claim 9, further comprising:
extracting, by the control system, a set of hemodynamic features from the second subset of detected heart rate waveforms; and
making, by the control system, a first blood pressure estimation based, at least in part, on the set of hemodynamic features.

13. The biometric method of claim 12, further comprising:
determining, by the control system, artery-vein phase shift, AVPS, data from the first subset of detected heart rate waveforms and the second subset of detected heart rate waveforms; and
making, by the control system, the first blood pressure estimation based, at least in part, on the AVPS data, the method optionally further comprising:
extracting, by the control system, heart rate waveform features from the heart rate waveforms;
making, by the control system, a second blood pressure estimation based, at least in part, on extracted heart rate waveform features; and
making, by the control system, a third blood pressure estimation based, at least in part, on the first blood pressure estimation and the second blood pressure estimation.

14. The biometric method of claim 9, further comprising:
determining, by the control system, artery-vein phase shift, AVPS, data from the heart rate waveforms; and
making, by the control system, a first blood pressure estimation based, at least in part, on the AVPS data7, the method optionally further comprising:
extracting, by the control system, heart rate waveform features from the heart rate waveforms;
making, by the control system, a second blood pressure estimation based, at least in part, on extracted heart rate waveform features; and
making, by the control system, a third blood pressure estimation based, at least in part, on the first blood pressure estimation and the second blood pressure estimation.

15. One or more non-transitory media having software stored thereon, the software including instructions for controlling the biometric system of claim 1 to perform the biometric method of any of claims 9 to 14.

## Patentansprüche

1. Biometrisches System, das Folgendes umfasst:
einen piezoelektrischen Empfänger;
ein Lichtquellensystem, konfiguriert zum Emittieren mehrerer Lichtimpulse mit einer Impulswiederholfrequenz zwischen 10 Hz und 1 MHz; und
ein Steuersystem, konfiguriert zum:
Steuern (305) des Lichtquellensystems zum Emittieren mehrerer Lichtimpulse in biologisches Gewebe mit der Impulswiederholfrequenz, wobei das biologische Gewebe Blut und Blutgefäße in Tiefen innerhalb des biologischen Gewebes umfasst;
Empfangen (310) von Signalen vom piezoelektrischen Empfänger entsprechend von Teilen des biologischen Gewebes emittierten akustischen Wellen, wobei die akustischen Wellen photoakustischen Emissionen aus dem Blut und den Blutgefäßen entsprechen, die durch die mehreren Lichtimpulse verursacht werden;
Erkennen (315) von Herzfrequenzwellenformen in den Signalen;
Bestimmen (320) eines ersten Teilsatzes von erkannten Herzfrequenzwellenformen entsprechend Venenherzfrequenzwellenformen; und
Bestimmen (325) eines zweiten Teilsatzes von erkannten Herzfrequenzwellenformen entsprechend Arterienherzfrequenzwellenformen,
wobei das Bestimmen des ersten und zweiten Teilsatzes von erkannten Herzfrequenzwellenformen als Venenherzfrequenzwellenformen bzw. Arterienherzfrequenzwellenformen das Bestimmen, nach einer Zeit jedes Lichtimpulses der mehreren Lichtimpulse, von Spitze-zu-Spitze-Werten der Signale während RGW (Range Gate Windows) mit derselben Zeitdauer, jedoch nach RGD (Range Gate Durations) unterschiedlicher Zeitdauer involviert.

2. Biometrisches System nach Anspruch 1, wobei das Steuersystem ferner konfiguriert ist zum:
Extrahieren von Herzfrequenzwellenformmerkmalen aus den Herzfrequenzwellenformen; und
Vornehmen einer Blutdruckschätzung zumindest teilweise auf der Basis von extrahierten Herzfrequenzwellenformmerkmalen.

3. Biometrisches System nach Anspruch 1, wobei das Empfangen der Signale von dem piezoelektrischen Empfänger das Erhalten von tiefendiskriminierten Signalen durch Anwenden erster bis *N-ter* Erfassungszeitverzögerungen und das Empfangen erster bis *N-ter* Signale während erster bis *N-ter* Erfassungszeitfenstern involviert, wobei jedes der ersten bis *N-ten* Erfassungszeitfenster nach einer entsprechenden einen der ersten bis *N-ten* Erfassungszeitverzögerungen auftritt, wobei *N* eine ganze Zahl größer als eins ist, wobei das Steuersystem zum Bestimmen des ersten Teilsatzes von erkannten Herzfrequenzwellenformen und des zweiten Teilsatzes von erkannten Herzfrequenzwellenformen zumindest teilweise auf der Basis der tiefendiskriminierten Signale konfiguriert ist.

4. Biometrisches System nach Anspruch 1, wobei das Steuersystem ferner konfiguriert ist zum:
Extrahieren eines Satzes von hämodynamischen Merkmalen aus dem zweiten Teilsatz von erkannten Herzfrequenzwellenformen; und
Vornehmen einer ersten Blutdruckschätzung zumindest teilweise auf der Satzes des Satzes von hämodynamischen Merkmalen.

5. Biometrisches System nach Anspruch 4, wobei das Steuersystem ferner konfiguriert ist zum:
Bestimmen von AVPS-(Artery-Vein Phase Shift)-Daten aus dem ersten Teilsatz von erkannten Herzfrequenzwellenformen und dem zweiten Teilsatz von erkannten Herzfrequenzwellenformen; und
Vornehmen der ersten Blutdruckschätzung zumindest teilweise auf der Basis der AVPS-Daten.

6. Biometrisches System nach Anspruch 5, wobei das Steuersystem ferner konfiguriert ist zum:
Extrahieren von Herzfrequenzwellenformmerkmalen aus den Herzfrequenzwellenformen;
Vornehmen einer zweiten Blutdruckschätzung zumindest teilweise auf der Basis von extrahierten Herzfrequenzwellenformmerkmalen; und
Vornehmen einer dritten Blutdruckschätzung zumindest teilweise auf der Basis der ersten Blutdruckschätzung und der zweiten Blutdruckschätzung.

7. Biometrisches System nach Anspruch 1, wobei das Steuersystem ferner konfiguriert ist zum:
Bestimmen von AVPS-(Artery-Vein Phase Shift)-Daten aus den Herzfrequenzwellenformen; und
Vornehmen einer ersten Blutdruckschätzung zumindest teilweise auf der Basis der AVPS-Daten.

8. Biometrisches System nach Anspruch 7, wobei das Steuersystem ferner konfiguriert ist zum:
Extrahieren von Herzfrequenzwellenformmerkmalen aus den Herzfrequenzwellenformen;
Vornehmen einer zweiten Blutdruckschätzung zumindest teilweise auf der Basis von extrahierten Herzfrequenzwellenformmerkmalen; und
Vornehmen einer dritten Blutdruckschätzung zumindest teilweise auf der Basis der ersten Blutdruckschätzung und der zweiten Blutdruckschätzung.

9. Biometrisches Verfahren, das Folgendes beinhaltet:
Steuern, über ein Steuersystem, eines Lichtquellensystems zum Emittieren mehrerer Lichtimpulse in biologisches Gewebe mit einer Impulswiederholfrequenz, wobei das biologische Gewebe Blut und Blutgefäße in Tiefen innerhalb des biologischen Gewebes umfasst;
Empfangen, durch das Steuersystem, von Signalen von einem piezoelektrischen Empfänger entsprechend von Teilen des biologischen Gewebes emittierten akustischen Wellen, wobei die akustischen Wellen photoakustischen Emissionen aus dem Blut und den Blutgefäßen entsprechen, die durch die mehreren Lichtimpulse verursacht werden;
Erkennen, durch das Steuersystem, von Herzfrequenzwellenformen in den Signalen;
Bestimmen, durch das Steuersystem, eines ersten Teilsatzes von erkannten Herzfrequenzwellenformen entsprechend Venenherzfrequenzwellenformen; und
Bestimmen, durch das Steuersystem, eines zweiten Teilsatzes von erkannten Herzfrequenzwellenformen entsprechend Arterienherzfrequenzwellenformen,
wobei das Bestimmen des ersten und zweiten Teilsatzes von erkannten Herzfrequenzwellenformen als Venenherzfrequenzwellenformen bzw. Arterienherzfrequenzwellenformen das Bestimmen, nach einer Zeit jedes Lichtimpulses der mehreren Lichtimpulse, von Spitze-zu-Spitze-Werten der Signale während RGW (Range Gate Windows) derselben Zeitdauer, jedoch nach RGD (Range Gate Durations) unterschiedlicher Zeitdauer involviert.

10. Biometrisches Verfahren nach Anspruch 9, das ferner Folgendes beinhaltet:
Extrahieren, durch das Steuersystem, von Herzfrequenzwellenformmerkmalen aus den Herzfrequenzwellenformen; und
Vornehmen, durch das Steuersystem, einer Blutdruckschätzung zumindest teilweise auf der Basis der extrahierten Herzfrequenzwellenformmerkmalen.

11. Biometrisches Verfahren nach Anspruch 9, wobei das Empfangen der Signale von dem piezoelektrischen Empfänger das Erhalten von tiefendiskriminierten Signalen durch Anwenden erster bis *N-ter* Erfassungszeitverzögerungen und das Empfangen erster bis *N-ter* Signale während erster bis *N-ter* Erfassungszeitfenstern involviert, wobei jedes der ersten bis *N-ten* Erfassungszeitfenster nach einer entsprechenden einen der ersten bis *N-ten* Erfassungszeitverzögerungen auftritt, wobei N eine ganze Zahl größer als eins ist, wobei das Verfahren ferner optional das Bestimmen, durch das Steuersystem, des ersten Teilsatzes von erkannten Herzfrequenzwellenformen und des zweiten Teilsatzes von erkannten Herzfrequenzwellenformen zumindest teilweise auf der Basis der tiefendiskriminierten Signale beinhaltet.

12. Biometrisches Verfahren nach Anspruch 9, das ferner Folgendes beinhaltet:
Extrahieren, durch das Steuersystem, eines Satzes von hämodynamischen Merkmalen aus dem zweiten Teilsatz von erkannten Herzfrequenzwellenformen; und
Vornehmen, durch das Steuersystem, einer ersten Blutdruckschätzung zumindest teilweise auf der Basis des Satzes von hämodynamischen Merkmalen.

13. Biometrisches Verfahren nach Anspruch 12, das ferner Folgendes beinhaltet:
Bestimmen, durch das Steuersystem, von AVPS-(Artery-Vein Phase Shift)-Daten aus dem ersten Teilsatz von erkannten Herzfrequenzwellenformen und dem zweiten Teilsatz von erkannten Herzfrequenzwellenformen; und
Vornehmen, durch das Steuersystem, der ersten Blutdruckschätzung zumindest teilweise auf der Basis der AVPS-Daten, wobei das Verfahren optional ferner Folgendes beinhaltet:
Extrahieren, durch das Steuersystem, von Herzfrequenzwellenformmerkmalen aus den Herzfrequenzwellenformen;
Vornehmen, durch das Steuersystem, einer zweiten Blutdruckschätzung zumindest teilweise auf der Basis von extrahierten Herzfrequenzwellenmerkmalen; und
Vornehmen, durch das Steuersystem, einer dritten Blutdruckschätzung zumindest teilweise auf der Basis der ersten Blutdruckschätzung und der zweiten Blutdruckschätzung.

14. Biometrisches Verfahren nach Anspruch 9, das ferner Folgendes beinhaltet:
Bestimmen, durch das Steuersystem, von AVPS-(Artery-Vein Phase Shift)-Daten aus den Herzfrequenzwellenformen; und
Vornehmen, durch das Steuersystem, einer ersten Blutdruckschätzung zumindest teilweise auf der Basis der AVPS-Daten, wobei das Verfahren optional ferner Folgendes beinhaltet:
Extrahieren, durch das Steuersystem, von Herzfrequenzwellenmerkmalen aus den Herzfrequenzwellenformen;
Vornehmen, durch das Steuersystem, einer zweiten Blutdruckschätzung zumindest teilweise auf der Basis von extrahierten Herzfrequenzwellenmerkmalen; und
Vornehmen, durch das Steuersystem, einer dritten Blutdruckschätzung zumindest teilweise auf der Basis der ersten Blutdruckschätzung und der zweiten Blutdruckschätzung.

15. Ein oder mehrere nichtflüchtige Medien, auf denen Software gespeichert ist, wobei die Software Befehle zum Steuern des biometrischen Systems nach Anspruch 1 zum Durchführen des biometrischen Verfahrens nach einem der Ansprüche 9 bis 14 enthält.

## Revendications

1. Système biométrique, comprenant :
un récepteur piézoélectrique ;
un système de source lumineuse configuré pour émettre une pluralité d'impulsions lumineuses à une fréquence de répétition d'impulsions comprise entre 10 Hz et 1 MHz ; et
un système de commande configuré pour :
commander (305) le système de source lumineuse pour émettre une pluralité d'impulsions lumineuses dans un tissu biologique à la fréquence de répétition d'impulsions, le tissu biologique comportant du sang et des vaisseaux sanguins à des profondeurs à l'intérieur du tissu biologique ;
recevoir (310) à partir du récepteur piézoélectrique des signaux correspondant à des ondes acoustiques émises par des parties du tissu biologique, les ondes acoustiques correspondant à des émissions photoacoustiques émanant du sang et des vaisseaux sanguins provoquées par la pluralité d'impulsions lumineuses ;
détecter (315) des formes d'onde de fréquence cardiaque dans les signaux ;
déterminer (320) un premier sous-ensemble de formes d'onde de fréquence cardiaque détectées correspondant à des formes d'onde de fréquence cardiaque veineuse ; et
déterminer (325) un second sous-ensemble de formes d'onde de fréquence cardiaque détectées correspondant à des formes d'onde de fréquence cardiaque artérielle,
dans lequel la détermination des premier et second sous-ensembles de formes d'onde de fréquence cardiaque détectées en tant que formes d'onde de fréquence cardiaque veineuse et formes d'onde de fréquence cardiaque artérielle, respectivement, implique de déterminer, après un temps de chaque impulsion lumineuse de la pluralité d'impulsions lumineuses, des valeurs crête à crête des signaux durant des fenêtres de distance (RGW) de même durée mais après des durées de fenêtres de distance (RGD) de durées différentes.

2. Système biométrique selon la revendication 1, dans lequel le système de commande est configuré en outre pour :
extraire des caractéristiques de forme d'onde de fréquence cardiaque à partir des formes d'onde de fréquence cardiaque ; et
réaliser une estimation de la pression artérielle sur la base, au moins en partie, de caractéristiques de forme d'onde de la fréquence cardiaque extraites.

3. Système biométrique selon la revendication 1, dans lequel la réception des signaux à partir du récepteur piézoélectrique implique l'obtention de signaux discriminés en profondeur en appliquant des 1 à *Nième* retards de temps d'acquisition et en recevant des 1 à *Nième* signaux durant des 1 à *Nième* fenêtres de temps d'acquisition, chacune des 1 à *Nième* fenêtres de temps d'acquisition se produisant après un retard correspondant des 1 à *Nième* retards de temps d'acquisition, dans lequel *N* est un entier supérieur à un, dans lequel le système de commande est configuré pour déterminer le premier sous-ensemble de formes d'onde de fréquence cardiaque détectées et le second sous-ensemble de formes d'onde de fréquence cardiaque détectées sur la base, au moins en partie, des signaux discriminés en profondeur.

4. Système biométrique selon la revendication 1, dans lequel le système de commande est configuré en outre pour :
extraire un ensemble de caractéristiques hémodynamiques à partir du second sous-ensemble de formes d'onde de fréquence cardiaque détectées ; et
réaliser une première estimation de pression artérielle sur la base, au moins en partie, de l'ensemble de caractéristiques hémodynamiques.

5. Système biométrique selon la revendication 4, dans lequel le système de commande est configuré en outre pour :
déterminer des données de déphasage artère-veine, AVPS, à partir du premier sous-ensemble de formes d'onde de fréquence cardiaque détectées et du second sous-ensemble de formes d'onde de fréquence cardiaque détectées ; et
réaliser la première estimation de pression artérielle sur la base, au moins en partie, des données AVPS.

6. Système biométrique selon la revendication 5, dans lequel le système de commande est configuré en outre pour :
extraire des caractéristiques de formes d'onde de fréquence cardiaque à partir des formes d'onde de fréquence cardiaque ;
réaliser une deuxième estimation de la pression artérielle sur la base, au moins en partie, de caractéristiques de forme d'onde de fréquence cardiaque extraites ; et
réalisation une troisième estimation de la pression artérielle sur la base, du moins en partie, de la première estimation de pression artérielle et la deuxième estimation de pression artérielle.

7. Système biométrique selon la revendication 1, dans lequel le système de commande est configuré en outre pour :
déterminer des données de déphasage artère-veine, AVPS, à partir des formes d'onde de fréquence cardiaque ; et
réaliser une première estimation de pression artérielle sur la base, au moins en partie, des données AVPS.

8. Système biométrique selon la revendication 7, dans lequel le système de commande est configuré en outre pour :
extraire des caractéristiques de formes d'onde de fréquence cardiaque à partir des formes d'onde de fréquence cardiaque ;
réaliser une deuxième estimation de pression artérielle sur la base, au moins en partie, de caractéristiques de forme d'onde de fréquence cardiaque extraites ; et
réaliser une troisième estimation de pression artérielle sur la base, au moins en partie, de la première estimation de pression artérielle et la deuxième estimation de pression artérielle.

9. Procédé biométrique, comprenant :
la commande, par l'intermédiaire d'un système de commande, d'un système de source lumineuse pour émettre une pluralité d'impulsions lumineuses dans un tissu biologique à une fréquence de répétition d'impulsions, le tissu biologique comportant du sang et des vaisseaux sanguins à des profondeurs à l'intérieur du tissu biologique ;
la réception, par le système de commande, de signaux à partir d'un récepteur piézoélectrique correspondant à des ondes acoustiques émises par des parties du tissu biologique, les ondes acoustiques correspondant à des émissions photoacoustiques émanant du sang et des vaisseaux sanguins provoquées par la pluralité d'impulsions lumineuses ;
la détection, par le système de commande, de formes d'onde de fréquence cardiaque dans les signaux ;
la détermination, par le système de commande, d'un premier sous-ensemble de formes d'onde de fréquence cardiaque détectées correspondant à des formes d'onde de fréquence cardiaque veineuse ; et
la détermination, par le système de commande, d'un second sous-ensemble de formes d'onde de fréquence cardiaque détectées correspondant à des formes d'onde de fréquence cardiaque artérielle,
dans lequel la détermination des premier et second sous-ensembles de formes d'onde de fréquence cardiaque détectées en tant que formes d'onde de fréquence cardiaque veineuse et formes d'onde de fréquence cardiaque artérielle, respectivement, implique de déterminer, après un temps de chaque impulsion lumineuse de la pluralité d'impulsions lumineuses, des valeurs crête à crête des signaux durant des fenêtres de distance (RGW) de même durée mais après des durées de fenêtres de distance (RGD) de durées différentes.

10. Procédé biométrique selon la revendication 9, comprenant en outre :
l'extraction, par le système de commande, de caractéristiques de forme d'onde de fréquence cardiaque à partir des formes d'onde de fréquence cardiaque ; et
la réalisation, par le système de commande, d'une estimation de pression artérielle sur la base, au moins en partie, de caractéristiques de forme d'onde de fréquence cardiaque extraites.

11. Procédé biométrique selon la revendication 9, dans lequel la réception des signaux à partir du récepteur piézoélectrique implique l'obtention de signaux discriminés en profondeur en appliquant des 1 à *Nième* retards de temps d'acquisition et en recevant des 1 à *Nième* signaux durant des 1 à *Nième* fenêtres de temps d'acquisition, chacune des 1 à *Nième* fenêtres de temps d'acquisition se produisant après un retard correspondant des 1 à *Nième* retards de temps d'acquisition, dans lequel *N* est un entier supérieur à un, le procédé comprenant éventuellement en outre la détermination, par le système de commande, du premier sous-ensemble de formes d'onde de fréquence cardiaque détectées et du second sous-ensemble de formes d'onde de fréquence cardiaque détectées sur la base, au moins en partie, des signaux discriminés en profondeur.

12. Procédé biométrique selon la revendication 9, comprenant en outre :
l'extraction, par le système de commande, d'un ensemble de caractéristiques hémodynamiques à partir du second sous-ensemble de formes d'onde de fréquence cardiaque détectées ; et
la réalisation, par le système de commande, d'une première estimation de pression artérielle sur la base, au moins en partie, de l'ensemble de caractéristiques hémodynamiques.

13. Procédé biométrique selon la revendication 12, comprenant en outre :
la détermination, par le système de commande, de données de déphasage artère-veine, AVPS, à partir du premier sous-ensemble de formes d'onde de fréquence cardiaque détectées et du second sous-ensemble de formes d'onde de fréquence cardiaque détectées ; et
la réalisation, par le système de commande, de la première estimation de pression artérielle sur la base, au moins en partie, des données AVPS, le procédé comprenant éventuellement en outre :
l'extraction, par le système de commande, de caractéristiques de forme d'onde de fréquence cardiaque à partir des formes d'onde de fréquence cardiaque ;
la réalisation, par le système de commande, d'une seconde estimation de pression artérielle sur la base, au moins en partie, des caractéristiques de forme d'onde de fréquence cardiaque extraites ; et
la réalisation, par le système de commande, d'une troisième estimation de pression artérielle sur la base, au moins en partie, de la première estimation de pression artérielle et de la deuxième estimation de pression artérielle.

14. Procédé biométrique selon la revendication 9, comprenant en outre :
la détermination, par le système de commande, de données de déphasage artère-veine, AVPS, des formes d'onde de fréquence cardiaque ; et
la réalisation, par le système de commande, d'une première estimation de pression artérielle sur la base, au moins en partie, des données AVPS, le procédé comprenant éventuellement en outre :
l'extraction, par le système de commande, de caractéristiques de forme d'onde de fréquence cardiaque à partir des formes d'onde de fréquence cardiaque ;
la réalisation, par le système de commande, d'une deuxième estimation de pression artérielle sur la base, au moins en partie, de caractéristiques de forme d'onde de fréquence cardiaque extraites ; et
la réalisation, par le système de commande, d'une troisième estimation de pression artérielle sur la base, au moins en partie, de la première estimation de pression artérielle et de la deuxième estimation de pression artérielle.

15. Un ou plusieurs supports non transitoires sur lesquels est stocké un logiciel, le logiciel comportant des instructions pour commander le système biométrique selon la revendication 1 afin de réaliser le procédé biométrique selon l'une quelconque des revendications 9 à 14.
